# EUROPEAN PATENT SPECIFICATION

(11) **EP 1 656 458 B1**
(45) Date of publication and mention of the grant of the patent: **20.05.2009**
(21) Application number: 04786410.3
(22) Date of filing: 20.08.2004
(51) Int. Cl.: C12Q 1/68

(54) **HUMAN AUTISM SUSCEPTIBILITY GENE AND USES THEREOF**
MENSCHLICHES AUTISMUS-SUSZEPTIBILITÄTSGEN UND VERWENDUNGEN DAVON
GENE HUMAIN DE SUSCEPTIBILITE A L'AUTISME ET SES UTILISATIONS

(30) Priority: 22.08.2003 US 496900 P; 22.08.2003 US 496917 P
(43) Date of publication of application: 17.05.2006
(73) Proprietor: Integragen, 91000 Evry (FR)
(72) Inventor: HAGER, Jörg, F-91540 Mennecy (FR); PHILIPPI, Anne, F-77310 St. Fargeau Ponthierry (FR); ROSCHMANN, Elke, F-91100 Corbeil Essonne (FR)
(74) Representative: Chajmowicz, Marion
(86) International application number: PCT/IB2004/002995
(87) International publication number: WO 2005/019474

(56) References cited:
- WO-A-00/68433
- WO-A-98/58082
- WO-A-99/55915
- WO-A-03/039342
- WO-A-03/045998
- DATABASE DBSNP [Online] NCBI; 20 October 2000 (2000-10-20), TSC-CSHL: "ss2372053 Fasta sequence" XP002323917 retrieved from HTTP://WWW.NCBI.NLM.NIH.GOV/SNP/SNP_SS.CGI ?SUBSNP_ID=2372053 Database accession no. SS2372053
- DATABASE DBSNP [Online] NCBI; 20 February 2004 (2004-02-20), CSHL-HUDD-: "ss19638392 Fasta sequence" XP002323918 retrieved from HTTP://WWW.NCBI.NLM.NIH.GOV/SNP/SNP_SS.CGI ?SUBSNP_ID=19638392 Database accession no. SS19638392
- LAMB JANINE A ET AL: "Autism: Recent molecular genetic advances" HUMAN MOLECULAR GENETICS, vol. 9, no. 6 Spec. Review Issue, 12 April 2000 (2000-04-12), pages 861-868, XP002323914 ISSN: 0964-6906
- PHILIPPE ANNE ET AL: "Genome-wide scan for autism susceptibility genes" HUMAN MOLECULAR GENETICS, vol. 8, no. 5, May 1999 (1999-05), pages 805-812, XP002323915 ISSN: 0964-6906
- TURNER MARTHA ET AL: "Genetic clues to the biological basis of autism" MOLECULAR MEDICINE TODAY, vol. 6, no. 6, June 2000 (2000-06), pages 238-244, XP002323919 ISSN: 1357-4310
- SHASTRY B S: "Molecular genetics of autism spectrum disorders" JOURNAL OF HUMAN GENETICS, vol. 48, no. 10, 2003, pages 495-501, XP002315920
- JAMAIN S ET AL: "Genetics of autism: From genome scans to candidate genes" MEDECINE/SCIENCES 2003 FRANCE, vol. 19, no. 11, 2003, pages 1081-1090, XP002323916 ISSN: 0767-0974

## Description

### FIELD OF THE INVENTION

The present invention relates generally to the fields of genetics and medicine. The present invention more particularly discloses the identification of a human autism susceptibility gene, which can be used for the diagnosis, prevention and treatment of autism, as well as for the screening of therapeutically active drugs. The invention more specifically discloses certain alleles of the solute carrier family 6 (neurotransmitter transporter, L-proline), member 7 or brain-specific L-proline transporter (SLC6A7) gene related to susceptibility to autism and representing novel targets for therapeutic intervention.

### BACKGROUND OF THE INVENTION

Autism is a neuropsychiatric developmental disorder characterized by impairments in reciprocal social interaction and verbal and non-verbal communication, restricted and stereotyped patterns of interests and activities, and the presence of developmental abnormalities by 3 years of age (Bailey et al., 1996). In his pioneer description of infantile autism, Kanner (1943) included the following symptoms: impaired language, lack of eye contact, lack of social interaction, repetitive behavior, and a rigid need for routine. He noted that in most cases the child's behavior was abnormal from early infancy. On this basis, he suggested the presence of an inborn, presumably genetic, defect. One year later, Hans Asperger in Germany described similar patients and termed the condition "autistic psychopathy".

Autism is defined using behavioral criteria because, so far, no specific biological markers are known for diagnosing the disease. The clinical picture of autism varies in severity and is modified by many factors, including education, ability and temperament. Furthermore, the clinical picture changes over the course of the development within an individual. In addition, autism is frequently associated with other disorders such as attention deficit disorder, motor incoordination and psychiatric symptoms such as anxiety and depression. There is some evidence that autism may also encompass epileptic, metabolic and immune disorder. In line with the clinical recognition of the variability, there is now general agreement that there is a spectrum of autistic disorders, which includes individuals at all levels of intelligence and language ability and spanning all degrees of severity.

Part of the autism spectrum, but considered a special subgroup, is Asperger syndrome (AS). AS is distinguished from autistic disorder by the lack of a clinically significant delay in language development in the presence of the impaired social interaction and restricted repetitive behaviors, interests, and activities that characterize the autism spectrum disorders (ASDs).

Pervasive developmental disorders (PPD) are also part of the ASDs. PPD is used to categorize children who do not meet the strict criteria for autism but who come close, either by manifesting atypical autism or by nearly meeting the diagnostic criteria in two or three of the key areas.

To standardize the diagnosis of autism, diagnostic criteria have been defined by the World Health Organisation (International Classification of Diseases, 10^{th} Revision (ICD-10), 1992) and the American Psychiatric Association (Diagnostic and Statistical Manual of Mental Disorders, 4^{th} edition (DSM-IV), 1994). An Autism Diagnostic Interview (ADI) has been developed (Le Couteur et al., 1989; Lord et al., 1994). The ADI is the only diagnostic tool available to diagnose ASD that has been standardized, rigorously tested and is universally recognized. The ADI is a scored, semistructured interview of parents that is based on ICD-10 and DSM-IV. criteria for the diagnosis of autism. It focuses on behavior in three main areas: qualities of reciprocal social interaction; communication and language; and restricted and repetitive, stereotyped interests and behaviors. Using these criteria, autism is no longer considered a rare disorder. Higher rates of 10-12 cases per 10,000 individuals have been reported in more recent studies (Gillberg and Wing, 1999) compared to the previously reported prevalence rate of 4-5 patients per 10,000 individuals based on Kanner's criteria (Folstein and Rosen-Sheidley, 2001). Estimates for the prevalence rate of the full spectrum of autistic disorders are 1.5 to 2.5 times higher. Reports of a four times higher occurrence in males compared to females are consistent. Mental retardation is present in between 25% and 40% of cases with ASD (Baird et al. 2000; Chakrabarti and Fombonne, 2001). Additional medical conditions involving the brain are seen in ca. 10% of the population (Gillberg and Coleman, 2000).

The mechanisms underlying the increase in reported cases of autism are unknown. It is highly debated whether this difference reflects an increase in the prevalence of autism, a gradual change in diagnostic criteria, a recognition of greater variability of disease expression, or an increased awareness of the disorder. In addition, there is a widespread public perception that the apparent increase is due primarily to environmentally factors (Nelson, 1991; Rodier and Hyman, 1998). However, it seems likely that most of the increased prevalence can be explained by a broadening of the diagnostic criteria, in combination with a broader application of these criteria.

Although there are effective treatments for ameliorating the disease, there are no cures available and benefits of treatment tend to be modest. Promising results have been obtained for several programs utilizing various behavioral and developmental strategies. Among the most promising are programs based on applied behavior analysis (ABA). Several medications appeared to improve various symptoms associated with autism, thereby increasing individuals' ability to benefit from educational and behavioral interventions. The most extensively studied agents are the dopamine antagonists. Several studies suggest the usefulness of various selective serotonin reuptake inhibitors.

Three twin studies have been performed to estimate heritability of autism (Folstein and Rutter, 1977; Bailey et al., 1995; Steffenburg et al., 1989). All twins who lived in a geographically defined population were sought out. In the combined data 36 monozygotic (MZ) and 30 dizygotic (DZ) twins were studied. The average MZ concordance rate is 70% compared to a DZ rate of 0%. A heritability of more than 90% was calculated from the MZ to DZ concordance ratio and the sibling recurrence risk that has been estimated to be ca 2%-4% (Jorde et al., 1991 Szatmari et al., 1998). Studies of non-autistic relatives have clearly shown that several characteristics of the ASDs are found more often in the parents of autistic children than the parents of controls including social reticence, communication difficulties, preference for routines and difficulty with change (Folstein and Rutter, 1977). Delayed onset of speech and difficulty with reading are also more common in family members of individuals with autism, as are recurrent depression, anxiety disorders, elevated platelet serotonin and increased head circumference (Folstein and Rosen-Sheidley, 2001).

The incidence of autism falls significantly with decreasing degree of relatedness to an affected individual indicating that a single-gene model is unlikely to account for most cases of autism (Jorde et al., 1990). A reported segregation analysis was most consistent with a polygenic mode of inheritance (Jorde et al., 1991). The most parsimonious genetic model is one in which several genes interact with one another to produce the autism phenotype (Folstein and Rosen-Sheidley, 2001).

Considerable indirect evidence indicates a possible role for autoimmunity in autism. One study found more family members with autoimmune diseases in families with an autistic proband compared with control probands (Comi et al., 1999). A few studies reported that haplotypes at the Moor Histocompatibility Complex (MHC) locus present in some children with autism, or their mothers, might predipose their autistic children to autoimmunity (Burger and Warren, 1998). In two studies, autoantibodies to certain brain tissues and proteins, including myelin basic protein, neurofilament proteins and vascular epithelium were found more often in autistic children compared to controls (Singh et al., 1993; Connolly et al., 1999; Weizman et al., 1982).

Although most autism cases are consistent with the proposed mechanism of oligogenicity and epistasis, a minority have been seen in association with chromosomal abnormalities and with disorders that have specific etiologies. Smalley (1997) stated that approximately 15 to 37% of cases of autism have a comorbid medical condition, including 5 to 14% with a known genetic disorder or chromosomal anomaly. Chromosome anomalies involving almost all human chromosomes have been reported. These include autosomal aneuploidies, sex-chromosome anomalies, deletions, duplications, translocations, ring chromosomes, inversions and marker chromosomes (Gillberg, 1998). Most common are abnormalities of the Prader Willi/Angelman Syndrome region on chromosome 15. Association of autism and a Mendelian condition or genetic syndrome included entreated phenylketonuria, fragile X syndrome, tuberous sclerosis and neurofibromatosis. Recently, Carney et al. (2003) identified mutations in the MECP2 (methyl CpG-binding protein 2) gene in two females with autism who do not have manifestations of Rett syndrome caused in 80% of the cases by mutations in the MECP2 gene.

Different groups are conducting genome scans related to autism or the broader phenotypes of ASDs. This approach appears very promising, because it is both systematic and model free. In addition, it has already been shown to be successful. Thus, positive linkage results have been obtained even by analysing comparatively small study groups. More important, some findings have already been replicated. The most consistent result was obtained for chromosome 7q, but there is also considerable overlap on chromosomes 2q and 16p (Folstein and Rosen-Sheidley, 2001). Considerable progress in identifying chromosomal regions have also been made on chromosome 15 and X. Mutations in two X-linked genes encoding neuroligins NLGN3 and NLGN4 have been identified in siblings with autism spectrum disorders (Jamain et al., 2003). Several lines of evidence support the fact that mutations in neuroligins are involved in autistic disorder. First, the reported mutations cause severe alterations of the predicted protein structure. Second, deletions at Xp22.3 that include NLGN34 have been reported in several autistic children. Third, a mutation in NLGN4 appeared *de novo* in one affected individual's mother.

WO 98/58 082 discloses a method for detecting the presence or predisposition to autism by detecting a polymorphism in a Hox gene.

### SUMMARY OF THE INVENTION

The present invention now discloses the identification of a human autism susceptibility gene, which can be used for the diagnosis, prevention and treatment of autism, as well as for the screening of therapeutically active drugs. The invention more specifically demonstrates that certain alleles of the SLC6A7 gene on chromosome 5 are related to susceptibility to autism and represent novel targets for therapeutic intervention. Any gene or protein involved in the regulation of the activity of SLC6A7, such as CAMK2, also represent novel targets for therapeutic intervention against autism.

The invention more specifically discloses certain alleles of the solute carrier family 6 (neurotransmitter transporter, L-proline), member 7 or brain-specific L-proline transporter (SLC6A7) gene related to susceptibility to autism and representing novel targets for therapeutic intervention. The present invention relates to particular mutations in the SLC6A7 gene and expression products, as well as to diagnostic tools and kits based on these mutations.

The invention can be used in the diagnosis of predisposition to or protection from, detection, prevention and/or treatment of autism, the method comprising detecting in a sample from the subject the presence of an alteration in the SLC6A7 gene or polypeptide, the presence of said alteration being indicative of the presence or predisposition to autism. The presence of said alteration can also be indicative for protecting from autism.

A particular, object of this invention resides in a method of detecting the presence of or predisposition to autism, in a subject, the method comprising detecting the presence of an alteration in the SLC6A7 gene locus in a sample from the subject, the presence of said alteration being indicative of the presence of or the predisposition to autism.

It is also described a method of detecting the protection from autism, in a subject, the method comprising detecting the presence of an alteration in the SLC6A7 gene locus in a sample from the subject, the presence of said alteration being indicative of the protection from autism.

It is further described in a method of assessing the response of a subject to a treatment of autism, the method comprising detecting the presence of an alteration in the SLC6A7 gene locus in a sample from the subject, the presence of said alteration being indicative of a particular response to said treatment.

It is also described in a method of assessing the adverse effect in a subject to a treatment of autism, the method comprising detecting the presence of an alteration in the SLC6A7 gene locus in a sample from the subject, the presence of said alteration being indicative of an adverse effect to said treatment.

It is further described a method for preventing autism in a subject, comprising detecting the presence of an alteration in the SLC6A7 gene locus in a sample from the subject, the presence of said alteration being indicative of the predisposition to autism and, administering a prophylactic treatment against autism.

The invention further relates to the screening of alteration(s) associated with autism in the SLC6A7 gene locus in patients. Such screenings are useful for diagnosing the presence, risk or predisposition to autism and/or for assessing the efficacy of a treatment of such disorders.

In a preferred embodiment, said alteration is one or several SNP(s) or a haplotype of SNPs associated with autism. More preferably, said haplotype associated with autism comprises or consists of several SNPs selected from the group consisting of SNP5, SNP6, SNP7, SNP8 and SNP10. Still more preferably, said haplotype is selected from the haplotypes disclosed in Table 4 and 5. More preferably, said SNP associated with autism can be SNP5, SNP6, SNP7, SNP8 and SNP 10.

Preferably, the alteration in the SLC5A7 gene locus is determined by performing a hydridization assay, a sequencing assay, a microsequencing assay, or an allele-specific amplification assay.

Compositions of matter are disclosed, comprising primers, probes, and/or oligonucleotides, which are designed to specifically detect at least one SNP or haplotype associated with autism in the genomic region including the SLC6A7 gene, or a combination thereof. More preferably, said haplotype associated with autism comprises or consists of several SNPs selected from the group consisting of SNP5, SNP6, SNP7, SNP8 and SNP10. Still more preferably, said haplotype is selected from the haplotypes disclosed in Table 4 and 5. More preferably, said SNP associated with autism can be SNP5, SNP6, SNP7, SNP8 and SNP10.

Methods of treating autism, in a subject through a modulation of SLC6A7 expression or activity are further disclosed. Such treatments use, for instance, SLC6A7 polypeptides, SLC6A7 DNA sequences (including antisense sequences directed at the SLC6A7 gene locus), anti-SLC6A7 antibodies or drugs that modulate SLC6A7 expression or activity.

Here are further described methods of treating individuals who carry deleterious alleles of the SLC6A7 gene, including pre-symptomatic treatment or combined therapy, such as through gene therapy, protein replacement therapy or through the administration of SLG6A7 protein mimetics and/or inhibitors.

A further aspect of this disclosure resides in the screening of drugs for therapy of autism, based on the modulation of or binding to an allele of SLC6A7 gene associated with autism or gene product thereof.

An additional aspect of this disclosure resides in the screening of drugs for therapy of autism based on the modulation of or binding to any gene or protein involved in the regulation of the activity of SLC6A7, preferably CAMK2.

The disclosure includes antibodies specific of an altered SLC6A7 polypeptide, fragments and derivatives of such antibodies, hybridomas secreting such antibodies, and diagnostic kits comprising those antibodies. More preferably, said antibodies are specific to a SLC6A7 polypeptide or a fragment thereof comprising an alteration, said alteration modifying the activity of SLC6A7.

It is further described a SLC6A7 gene or a fragment thereof comprising an alteration, said alteration modifying the activity of SLC6A7. The invention further concerns a SLC6A7 polypeptide or a fragment thereof comprising an alteration, said alteration modifying the activity of SLC6A7.

### LEGEND TO THE FIGURES

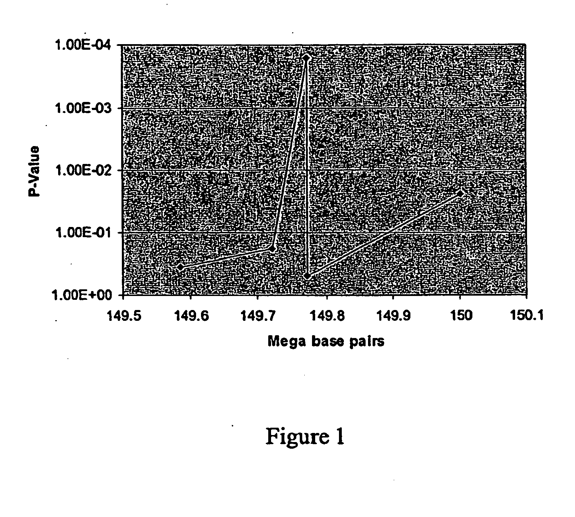

Figure 1: High density mapping using Genomic Hybrid Identity Profiling (GenomeHTP)
   A total of 2263 BAC clones with an average spacing of 1.2 Mega base pairs between clones representing the whole human genome were tested for linkage using GenomeHIP. Each point on the x-axis corresponds to a clone. Several clones are indicated by their library name for better orientation (e.g. FEODBACA19ZG04). Suggestive evidence for linkage was calculated for clone FE0DBACA28QZH06 (p-value 1.64 x 10⁴) encompassing a region starting from 149772850 base pairs to 149921225 base pairs on human chromosome 5. The p-value 7.0 x 10⁻⁴ corresponding to the significance level for suggestive linkage proposed by Lander and Kruglyak (1995) for whole genome screens was used as a significance level.

### DETAILED DESCRIPTION OF THE INVENTION

The present invention discloses the identification ofSLC6A7 as a human autism susceptibility gene. Various nucleic acid samples from 114 families with autism were submitted to a particular GenomeHIP process. This process led to the identification of particular identical-by-descent fragments in said populations that are altered in autistic subjects. By screening of the IBD fragments including the immediate neighbouring regions, we identified the solute carrier family 6 (neurotransmitter transporter, L-proline), member 7 (SLC6A7) on chromosome 5q31-32 gene as a candidate for autism and related phenotypes. This gene is indeed present in the interval and expresses a functional phenotype consistent with a genetic regulation of autism.

The present invention thus proposes to use SLC6A7 gene and corresponding expression products for the diagnosis, prevention and treatment of autism as well as for the screening of therapeutically active drugs.

### DEFINITIONS

Autism and autism spectrum disorders (ASDs): Autism is typically characterized as part of a spectrum of disorders (ASDs) including Asperger syndrome (AS) and other pervasive developmental disorders (PPD). Autism shall be construed as any condition of impaired social interaction and communication with restricted repetitive and stereotyped patterns of behavior, interests and activities present before the age of 3, to the extent that health may be impaired. AS is distinguished from autistic disorder by the lack of a clinically significant delay in language development in the presence of the impaired social interaction and restricted repetitive behaviors, interests, and activities that characterize the autism-spectrum disorders (ASDs). PPD is used to categorize children who do not meet the strict criteria for autism but who come close, either by manifesting atypical autism or by nearly meeting the diagnostic criteria in two or three of the key areas.

The invention may be used in various subjects, particularly human, including adults, children and at the prenatal stage.

Within the context of this invention, the SLC6A7 gene locus designates all SLC6A7 sequences or products in a cell or organism, including SLC6A7 coding sequences, SLC6A7 non-coding sequences (e.g., introns), SLC6A7 regulatory sequences controlling transcription and/or translation (e.g., promoter, enhancer, terminator, etc.), as well as all corresponding expression products, such as SLC6A7 RNAs (e.g., mRNAs) and SLC6A7 polypeptides (e.g., a pre-protein and a mature protein). The SLC6A7 gene locus also comprise surrounding sequences of the SLC6A7 gene which include SNPs that are in linkage disequilibrium with SNPs located in the SLC6A7 gene. For example, the SLC6A7 locus comprises surrounding sequences comprising SNP3, SNP4, SNP5 and SNP14.

As used in the present application, the term "SLC6A7 gene," designates the solute carrier family 6 (neurotransmitter transporter, L-proline), member 7 or brain-specific L-proline transporter gene on human chromosome 5, as well as variants, analogs and fragments thereof, including alleles thereof (e.g., germline mutations) which are related to susceptibility to autism. The SLC6A7 gene may also be referred to as PROT.

The term "gene" shall be construed to include any type of coding nucleic acid, including genomic DNA (gDNA), complementary DNA (cDNA), synthetic or semi-synthetic DNA, as well as any form of corresponding RNA. The term gene particularly includes recombinant nucleic acids encoding SLC6A7, i.e., any non naturally occurring nucleic acid molecule created artificially, e.g., by assembling, cutting, ligating or amplifying sequences. A SLC6A7 gene is typically double-stranded, although other forms may be contemplated, such as single-stranded. SLC6A7 genes may be obtained from various sources and according to various techniques known in the art, such as by screening DNA libraries or by amplification from various natural sources. Recombinant nucleic acids may be prepared by conventional techniques, including chemical synthesis, genetic engineering, enzymatic techniques, or a combination thereof. Suitable SLC6A7 gene sequences may be found on gene banks, such as Unigene Cluster for SLC6A7 (Hs. 241597) or NCBI Reference Sequences (NM_014228). A particular example of a SLC6A7 gene comprises SEQ ID No: 1.

The term "SLC6A7 gene" includes any variant, fragment or analog of SEQ ID No:1 or of any coding sequence as identified above. Such variants include, for instance, naturally-occurring variants due to allelic variations between individuals (e.g., polymorphism), mutated alleles related to autism, alternative splicing forms, etc. The term variant also includes SLC6A7 gene sequences from other sources or organisms. Variants are preferably substantially homologous to SEQ ID No: 1, i.e., exhibit a nucleotide sequence identity of at least about 65%, typically at least about 75%, preferably at least about 85%, more preferably at least about 95% with SEQ ID No: 1. Variants and analogs of a SLC6A7 gene also include nucleic acid sequences, which hybridize to a sequence as defined above (or a complementary strand thereof) under stringent hybridization conditions.

Typical stringent hybridisation conditions include temperatures above 30° C, preferably above 35°C, more preferably in excess of 42°C, and/or salinity of less than about 500 mM, preferably less than 200 mM. Hybridization conditions may be adjusted by the skilled person by modifying the temperature, salinity and/or the concentration of other reagents such as SDS, SSC, etc.

A fragment of a SLC6A7 gene designates any portion of at least about 8 consecutive nucleotides of a sequence as disclosed above, preferably at least about 15, more preferably at least about 20 nucleotides, further preferably of at least 30 nucleotides. Fragments include all possible nucleotide length between 8 and 100 nucleotides, preferably between 15 and 100, more preferably between 20 and 100.

A SLC6A7 polypeptide designates any protein or polypeptide encoded by a SLC6A7 gene as disclosed above. The term "polypeptide," refers to any molecule comprising a stretch of amino acids. This term includes molecules of various length, such as peptides and proteins. The polypeptide may be modified, such as by glycosylations and/or acetylations and/or chemical reaction or coupling, and may contain one or several non-natural or synthetic amino acids. A specific example of a SLC6A7 polypeptide comprises all or part of SEQ ID No:2.

As used in the present application, the term "CAMK2A gene" designates the calcium/calmodulin-dependent protein kinase (CaM kinase) II alpha gene on human chromosome 5, as well as variants, analogs and fragments thereof. The CAMK2A gene may also be referred to as CAMKA, KIAA0968. The term "gene" shall be construed to include any type of coding nucleic acid, including genomic DNA (gDNA), complementary DNA (cDNA), synthetic or semi-synthetic DNA, as well as any form of corresponding RNA. The term gene particularly includes recombinant nucleic acids encoding CAMK2A, i.e., any non naturally occurring nucleic acid molecule created artificially, e.g., by assembling, cutting, ligating or amplifying sequences. A CAMK2A gene is typically double-stranded, although other forms may be contemplated, such as single-stranded. CAMK2A genes may be obtained from various sources and according to various techniques known in the art, such as by screening DNA libraries or by amplification from various natural sources. Recombinant nucleic acids may be prepared by conventional techniques, including chemical synthesis, genetic engineering, enzymatic techniques, or a combination thereof. Suitable CAMK2A gene sequences may be found on gene banks, such as Unigene Cluster for CAMK2A (Hs. 143535) or NCBI Reference Sequences (NM_015981 and NM_171825).

A fragment of a CAMK2A gene designates any portion of at least about 8 consecutive nucleotides of a sequence as disclosed above, preferably at least about 15, more preferably at least about 20 nucleotides, further preferably of at least 30 nucleotides. Fragments include all possible nucleotide length between 8 and 100 nucleotides, preferably between 15 and 100, more preferably between 20 and 100.

A CAMK2A polypeptide designates any protein or polypeptide encoded by a CAMK2A gene as disclosed above. The term "polypeptide" refers to any molecule comprising a stretch of amino acids. This term includes molecules of various length, such as peptides and proteins. The polypeptide may be modified, such as by glycosylations and/or acetylations and/or chemical reaction or coupling, and may contain one or several non-natural or synthetic amino acids. A specific example of a CAMK2A polypeptide comprises all or part of the sequence NP_057065 or a variant thereof.

The terms "response to a treatment" refer to treatment efficacy, including but not limited to ability to metabolise a therapeutic compound, to the ability to convert a pro-drug to an active drug, and to the pharmacokinetics (absorption, distribution, elimination) and the pharmacodynamics (receptor-related) of a drug in an individual.

The terms "adverse effects to a treatment" refer to adverse effects of therapy resulting from extensions of the principal pharmacological action of the drug or to idiosyncratic adverse reactions resulting from an interaction of the drug with unique host factors. "Side effects to a treatment" include, but are not limited to, adverse reactions such as dermatologic, hematologic or hepatologic toxicities and further includes gastric and intestinal ulceration, disturbance in platelet function, renal injury, generalized urticaria, bronchoconstriction, hypotension, and shock.

### DIAGNOSIS

The invention now provides diagnosis methods based on a monitoring of the SLC6A7 gene locus in a subject. Within the context of the present invention, the term 'diagnosis" includes the detection, monitoring, dosing, comparison, etc., at various stages, including early, pre-symptomatic stages, and late stages, in adults, children and pre-birth. Diagnosis typically includes the prognosis, the assessment of a predisposition or risk of development, the characterization of a subject to define most appropriate treatment (pharmaco-genetics), etc.

A particular object of this invention resides in a method of detecting the presence of or predisposition to autism in a subject, the method comprising detecting in a sample from the subject the presence of an alteration in the SLC6A7 gene locus in said sample. The presence of said alteration is indicative of the presence or predisposition to autism. Preferably, said alteration is selected from a group consisting of SNPs in the gene or a combination thereof. Optionally, said method comprises a previous step of providing a sample from a subject. Preferably, the presence of an alteration in the SLC6A7 gene locus in said sample is detected through the genotyping of a sample.

Another particular object of this invention resides in a method of detecting the protection from autism, in a subject, the method comprising detecting the presence of an alteration in the SLC6A7 gene locus in a sample from the subject, the presence of said alteration being indicative of the protection from autism.

It is further described a method of assessing the response of a subject to a treatment of autism, the method comprising detecting in a sample from the subject the presence of an alteration in the SLC6A7 gene locus in said sample. The presence of said alteration is indicative of a particular response to said treatment. Preferably, said alteration is selected from a group consisting of SNPs in the gene or a combination thereof. Optionally, said method comprises a previous step of providing a samples from a subject. Preferably, the presence of an alteration in the SLC6A7 gene locus in said sample is detected through the genotyping of a sample.

It is also described a method of assessing the adverse effects of a subject to a treatment of autism, the method comprising detecting in a sample from the subject the presence of an alteration in the SLC6A7 gene locus in said sample. The presence of said alteration is indicative of adverse effects to said treatment. Preferably, the presence of an alteration in the SLC6A7 gene locus in said sample is detected through the genotyping of a sample.

It is further described a method for preventing autism in a subject, comprising detecting the presence of an alteration in the SLC6A7 gene locus in a sample from the subject, the presence of said alteration being indicative of the predisposition to autism; and, administering a prophylactic treatment against autism. Said prophylactic treatment can be a drug administration.

In the diagnostic / detection methods of this invention, any alteration in the SLC6A7 locus may be assessed in combination with other markers such as other alterations in any other gene or protein.

Diagnostics, which analyse and predict response to a treatment or drug, or side effects to a treatment or drug, may be used to determine whether an individual should be treated with a particular treatment drug. For example, if the diagnostic indicates a likelihood that an individual will respond positively to treatment with a particular drug, the drug may be administered to the individual. Conversely, if the diagnostic indicates that an individual is likely to respond negatively to treatment with a particular drug, an alternative course of treatment may be prescribed. A negative response may be defined as either the absence of an efficacious response or the presence of toxic side effects.

Clinical drug trials represent another application for the present invention. One or more SLC6A7 SNPs indicative of response to a drug or to side effects to a drug may be identified using the methods described above. Thereafter, potential participants in clinical trials of such an agent may be screened to identify those individuals most likely to respond favorably to the drug and exclude those likely to experience side effects. In that way, the effectiveness of drug treatment may be measured in individuals who respond positively to the drug, without lowering the measurement as a result of the inclusion of individuals who are unlikely to respond positively in the study and without risking undesirable safety problems.

The alteration may be determined at the level of the SLC6A7 gDNA, RNA or polypeptide. Optionally, the detection is performed by sequencing all or part of the SLC6A7 gene or by selective hybridisation or amplification of all or part of the SLC6A7 gene. More preferably a SLC6A7 gene specific amplification is carried out before the alteration identification step.

An alteration in the SLC6A7 gene locus may be any form of mutation(s), deletion(s), rearrangement(s) and/or insertions in the coding and/or non-coding region of the locus, alone or in various combination(s). Mutations more specifically include point mutations. Deletions may encompass any region of two or more residues in a coding or non-coding portion of the gene locus, such as from two residues up to the entire gene or locus. Typical deletions affect smaller regions, such as domains (introns) or repeated sequences or fragments of less than about 50 consecutive base pairs, although larger deletions may occur as well. Insertions may encompass the addition of one or several residues in a coding or non-coding portion of the gene locus. Insertions may typically comprise an addition of between 1 and 50 base pairs in the gene locus. Rearrangement includes inversion of sequences. The SLC6A7 gene locus alteration may result in the creation of stop codons, frameshift mutations, amino acid substitutions, particular RNA splicing or processing, product instability, truncated polypeptide production, etc. The alteration may result in the production of a SLC6A7 polypeptide with altered function, stability, targeting or structure. The alteration may also cause a reduction in protein expression or, alternatively, an increase in said production.

In a particular embodiment of the method according to the present invention, the alteration in the SLC6A7 gene locus is selected from a point mutation, a deletion and an insertion in the SLC6A7 gene or corresponding expression product, more preferably a point mutation and a deletion. The alteration may be determined at the level of the SLC6A7 gDNA, RNA or polypeptide.

In this regard, the present invention now discloses SNPs in the SLC6A7 gene and certain haplotypes, which include SNPs selected from the group consisting of SNP3, SNP4, SNP5, SNP6, SNP7, SNP8, SNP9, SNP10, SNP12 and SNP14 that are associated with autism. The SNPs are reported in the following Table 1.

**Table 1**

| Nucleotide position in genomic sequence of chromosome (Build34) | SNP identity | dbSNP reference | Polymorphism | Position in locus and type of amino acid change | Position in SEQ ID NO: 1 |
|---|---|---|---|---|---|
| 149023175 | SNP3 | rs1531236 | C/T | 5' of SLC6A7 locus | |
| 149037031 | SNP4 | rs3733660, rs1135093 | A/G | 5' of SLC6A7 locus | |
| 149594018 | SNP5 | rs6890699 | C/G | 5' of SLC6A7 locus | |
| 149598218 | SNP6 | rs3764886 | A/G | 5'UTR | 289 |
| 149599057 | SNP7 | rs758590 | C/T | intron | 1128 |
| 149601459 | SNP8 | rs917585 | C/G | intron | 3530 |
| 149604212 | SNP9 | rs2240784 | C/T | intron | 5421 |
| 149606429 | SNP10 | rs758593 | A/G | intron | 8500 |
| 149613943 | SNP12 | rs3815375 | A/G | intron | 16014 |
| 149659923 | SNP14 | rs2288799 | A/G | 3' of SLC6A7 locus | |

In a preferred embodiment, the alteration is one or several SNP(s) or a haplotype of SNPs associated with autism. More preferably, said haplotype associated with autism comprises or consists of several SNPs selected from the group consisting of SNP5, SNP6, SNP7, SNP8 and SNP10. Still more preferably, said haplotype is selected from the haplotypes disclosed in Table 4 and 5. More preferably, said SNP associated with autism can be SNP5, SNP6, SNP7, SNP8 and SNP10.

Preferably, the alteration in the SLC6A7 gene locus is determined by performing a hydridization assay, a sequencing assay, a microsequencing assay, or an allele-specific amplification assay.

In any method according to the present invention, one or several SNPs in the SLC6A7 gene and certain haplotypes comprising SNPs in the SLC6A7 gene, more particularly SNPS, SNP6, SNP7, SNP8and SNP10, can be used in combination with other SNPs or haplotypes associated with autism. These SNPs can also be combined with SNPs located in other gene(s).

In a first variant, the method of the present invention comprises detecting the presence of an altered SLC6A7 gene sequence. This can be performed by sequencing all or part of the SLC6A7 gene, polypeptide or RNA, by selective hybridisation or by selective amplification, for instance.

A more specific embodiment comprises detecting the presence of at least one SNP in the SLC6A7 gene sequence of a subject, or any combination thereof.

In another variant, the method comprises detecting the presence of an altered SLC6A7 RNA expression. Altered RNA expression includes the presence of an altered RNA sequence, the presence of an altered RNA splicing or processing, the presence of an altered quantity of RNA, etc. These may be detected by various techniques known in the art, including by sequencing all or part of the SLC6A7 RNA or by selective hybridisation or selective amplification of all or part of said RNA, for instance.

In a further variant, the method comprises detecting the presence of an altered SLC6A7 polypeptide expression. Altered SLC6A7 polypeptide expression includes the presence of an altered polypeptide sequence, the presence of an altered quantity of SLC6A7 polypeptide, the presence of an altered tissue distribution, etc. These may be detected by various techniques known in the art, including by sequencing and/or binding to specific ligands (such as antibodies), for instance.

As indicated above, various techniques known in the art may be used to detect or quantify altered SLC6A7 gene or RNA expression or sequence, including sequencing, hybridisation, amplification and/or binding to specific ligands (such as antibodies). Other suitable methods include allele-specific oligonucleotide (ASO), allele-specific amplification, Southern blot (for DNAs), Northern blot (for RNAs), single-stranded conformation analysis (SSCA), PFGE, fluorescent in situ hybridization (FISH), gel migration, clamped denaturing gel electrophoresis, heteroduplex analysis, RNase protection, chemical mismatch cleavage, ELISA, radio-immunoassays (RIA) and immuno-enzymatic assays (IEMA).

Some of these approaches (e.g., SSCA and CGGE) are based on a change in electrophoretic mobility of the nucleic acids, as a result of the presence of an altered sequence. According to these techniques, the altered sequence is visualized by a shift in mobility on gels. The fragments may then be sequenced to confirm the alteration.

Some others are based on specific hybridisation between nucleic acids from the subject and a probe specific for wild-type or altered SLC6A7 gene or RNA. The probe may be in suspension or immobilized on a substrate. The probe is typically labelled to facilitate detection of hybrids.

Some of these approaches are particularly suited for assessing a polypeptide sequence or expression level, such as Northern blot, ELISA and RIA. These latter require the use of a ligand specific for the polypeptide, more preferably of a specific antibody.

In a particular, preferred, embodiment, the method comprises detecting the presence of an altered SLC6A7 gene expression profile in a sample from the subject. As indicated above, this can be accomplished more preferably by sequencing, selective hybridisation and/or selective amplification of nucleic acids present in said sample.

### Sequencing

Sequencing can be carried out using techniques well known in the art, using automatic sequencers. The sequencing may be performed on the complete SLC6A7 gene or, more preferably, on specific domains thereof, typically those known or suspected to carry deleterious mutations or other alterations.

### Amplification

Amplification is based on the formation of specific hybrids between complementary nucleic acid sequences that serve to initiate nucleic acid reproduction.

Amplification may be performed according to various techniques known in the art, such as by polymerase chain reaction (PCR), ligase chain reaction (LCR), strand displacement amplification (SDA) and nucleic acid sequence based amplification (NASBA). These techniques can be performed using commercially available reagents and protocols. Preferred techniques use allele-specific PCR or PCR-SSCP. Amplification usually requires the use of specific nucleic acid primers, to initiate the reaction.

Nucleic acid primers useful for amplifying sequences from the SLC6A7 gene or locus are able to specifically hybridize with a portion of the SLC6A7 gene locus that flank a target region of said locus, said target region being altered in certain subjects having autism.

Primers that can be used to amplify SLC6A7 target region may be designed based on the genomic or RNA sequence of SLC6A7 and, in particular, on the sequence of SEQ ID No: 1.

Another particular object of this disclosure resides in a nucleic acid primer useful for amplifying sequences from the SLC6A7 gene or locus including surrounding regions. Such primers are preferably complementary to, and hybridize specifically to nucleic acid sequences in the SLC6A7 gene locus. Particular primers are able to specifically hybridise with a portion of the SLC6A7 gene locus that flank a target region of said locus, said target region being altered in certain subjects having autism.

The disclosure also relates to a nucleic acid primer, said primer being complementary to and hybridizing specifically to a portion of a SLC6A7 coding sequence (e.g., gene or RNA) altered in certain subjects having autism. In this regard, particular primers of this invention are specific for altered sequences in a SLC6A7 gene or RNA. By using such primers, the detection of an amplification product indicates the presence of an alteration in the SLC6A7 gene locus. In contrast, the absence of amplification product indicates that the specific alteration is not present in the sample.

Typical primers are single-stranded nucleic acid molecules of about 5 to 60 nucleotides in length, more preferably of about 8 to about 25 nucleotides in length. The sequence can be derived directly from the sequence of the SLC6A7 gene locus. Perfect complementarity is preferred, to ensure high specificity. However, certain mismatch may be tolerated.

Also described is the use of a nucleic acid primer or a pair of nucleic acid primers as described above in a method of detecting the presence of or predisposition to autism in a subject or in a method of assessing the response of a subject to a treatment of autism.

### Selective hybridization

Hybridization detection methods are based on the formation of specific hybrids between complementary nucleic acid sequences that serve to detect nucleic acid sequence alteration(s).

A particular detection technique involves the use of a nucleic acid probe specific for wild-type or altered SLC6A7 gene or RNA, followed by the detection of the presence of a hybrid. The probe may be in suspension or immobilized on a substrate or support (as in nucleic acid array or chips technologies). The probe is typically labelled to facilitate detection of hybrids.

In this regard, a particular embodiment of this invention comprises contacting the sample from the subject with a nucleic acid probe specific for an altered SLC6A7 gene locus, and assessing the formation of an hybrid. In a particular, preferred embodiment, the method comprises contacting simultaneously the sample with a set of probes that are specific, respectively, for wild type SLC6A7 gene locus and for various altered forms thereof. In this embodiment, it is possible to detect directly the presence of various forms of alterations in the SLC6A7 gene locus in the sample. Also, various samples from various subjects may be treated in parallel.

Within the context of this invention, a probe refers to a polynucleotide sequence which is complementary to and capable of specific hybridisation with a (target portion of a) SLC6A7 gene or RNA, and which is suitable for detecting polynucleotide polymorphisms associated with SLC6A7 alleles which predispose to or are associated with autism. Probes are preferably perfectly complementary to the SLC6A7 gene, RNA, or target portion thereof. Probes typically comprise single-stranded nucleic acids of between 8 to 1000 nucleotides in length, for instance of between 10 and 800, more preferably of between 15 and 700, typically of between 20 and 500. It should be understood that longer probes may be used as well. A preferred probe of this invention is a single stranded nucleic acid molecule of between 8 to 500 nucleotides in length, which can specifically hybridise to a region of a SLC6A7 gene or RNA that carries an alteration.

A specific embodiment of this disclosure is a nucleic acid probe specific for an altered (e.g., a mutated) SLC6A7 gene or RNA, i.e., a nucleic acid probe that specifically hybridises to said altered SLC6A7 gene or RNA and essentially does not hybridise to a SLC6A7 gene or RNA lacking said alteration. Specificity indicates that hybridisation to the target sequence generates a specific signal which can be distinguished from the signal generated through non-specific hybridisation. Perfectly complementary sequences are preferred to design probes according to this invention. It should be understood, however, that certain mismatch may be tolerated, as long as the specific signal may be distinguished from non-specific hybridisation.

Particular examples of such probes are nucleic acid sequences complementary to a target portion of the SLC6A7 gene or RNA carrying a point mutation as listed in Table 1 above. More particularly, the probes can comprise a sequence selected from the group consisting of SEQ ID NOs 3-12 or a fragment thereof comprising the SNP or a complementary sequence thereof.

The sequence of the probes can be derived from the sequences of the SLC6A7 gene and RNA as provided in the present application. Nucleotide substitutions may be performed, as well as chemical modifications of the probe. Such chemical modifications may be accomplished to increase the stability of hybrids (e.g., intercalating groups) or to label the probe. Typical examples of labels include, without limitation, radioactivity, fluorescence, luminescence, enzymatic labelling, etc.

The disclosure also concerns the use of a nucleic acid probe as described above in a method of detecting the presence of or predisposition to autism in a subject or in a method of assessing the response of a subject to a treatment of autims.

### Specific Ligand Binding

As indicated above, alteration in the SLC6A7 gene locus may also be detected by screening for alteration(s) in SLC6A7 polypeptide sequence or expression levels. In this regard, a specific embodiment of this invention comprises contacting the sample with a ligand specific for a SLC6A7 polypeptide and determining the formation of a complex.

Different types of ligands may be used, such as specific antibodies. In a specific embodiment, the sample is contacted with an antibody specific for a SLC6A7 polypeptide and the formation of an immune complex is determined. Various methods for detecting an immune complex can be used, such as ELISA, radio-immunoassays (RIA) and immuno-enzymatic assays (IEMA).

Within the context of this disclosure an antibody designates a polyclonal antibody, a monoclonal antibody, as well as fragments or derivatives thereof having substantially the same antigen specificity. Fragments include Fab, Fab'2, CDR regions, etc. Derivatives include single-chain antibodies, humanized antibodies, poly-functional antibodies, etc.

An antibody specific for a SLC6A7 polypeptide designates an antibody that selectively binds a SLC6A7 polypeptide, i.e., an antibody raised against a SLC6A7 polypeptide or an epitope-containing fragment thereof. Although non-specific binding towards other antigens may occur, binding to the target SLC6A7 polypeptide occurs with a higher affinity and can be reliably discriminated from non-specific binding.

In a specific embodiment, the method comprises contacting a sample from the subject with (a support coated with) an antibody specific for an altered form of a SLC6A7 polypeptide, and determining the presence of an immune complex. In a particular embodiment, the sample may be contacted simultaneously, or in parallel, or sequentially, with various (supports coated with) antibodies specific for different forms of a SLC6A7 polypeptide, such as a wild-type and various altered forms thereof.

It is further described the use of a ligand, preferably an antibody, a fragment or a derivative thereof as described above, in a method of detecting the presence of or predisposition to autism, in a subject or in a method of assessing the response of a subject to a treatment of autism.

It is also described a diagnostic kit comprising products and reagents for detecting in a sample from a subject the presence of an alteration in the SLC6A7 gene or polypeptide, in the SLC6A7 gene or polypeptide expression, and/or in SLC6A7 activity. Said diagnostic, kit comprises any primer, any pair of primers, any nucleic acid probe and/or any ligand, preferably antibody, described in the present invention. Said diagnostic kit according to the present invention can further comprise reagents and/or protocols for performing a hybridization, amplification or antigen-antibody immune reaction.

The diagnosis methods can be performed in vitro or ex vivo. They use a sample from the subject, to assess the status of the SLC6A7 gene locus. The sample may be any biological sample derived from a subject, which contains nucleic acids or polypeptides. Examples of such samples include fluids, tissues, cell samples, organs, biopsies, etc. Most preferred samples are blood, plasma, saliva, urine, seminal fluid, etc. Pre-natal diagnosis may also be performed by testing foetal cells or placental cells, for instance The sample may be collected according to conventional techniques, including non-invasive techniques, and used directly for diagnosis or stored, or obtained from any sample collections. The sample may be treated prior to performing the method, in order to render or improve availability of nucleic acids or polypeptides for testing. Treatments include, for instant, lysis (e.g., mechanical, physical, chemical, etc.), centrifugation, etc. Also, the nucleic acids and/or polypeptides may be pre-purified or enriched by conventional techniques, and/or reduced in complexity. Nucleic acids and polypeptides may also be treated with enzymes or other chemical or physical treatments to produce fragments thereof. Considering the high sensitivity of the claimed methods, very few amounts of sample are sufficient to perform the assay.

As indicated, the sample is preferably contacted with reagents such as probes, primers or ligands in order to assess the presence of an altered SLC6A7 gene locus. Contacting may be performed in any suitable device, such as a plate, tube, well, glass, etc. In specific embodiments, the contacting is performed on a substrate coated with the reagent, such as a nucleic acid array or a specific ligand array. The substrate may be a solid or semi-solid substrate such as any support comprising glass, plastic, nylon, paper, metal, polymers and the like. The substrate may be of various forms and sizes, such as a slide, a membrane, a bead, a column, a gel, etc. The contacting may be made under any condition suitable for a complex to be formed between the reagent and the nucleic acids or polypeptides of the sample.

The finding of an altered SLC6A7 polypeptide, RNA or DNA in the sample is indicative of the presence of an altered SLC6A7 gene locus in the subject, which can be correlated to the presence, predisposition or stage of progression of autism. For example, an individual having a germline SLC6A7 mutation has an increased risk of developing autism. The determination of the presence of an altered SLC6A7 gene locus in a subject also allows the design of appropriate therapeutic intervention, which is more effective and customized. Also, this determination at the pre-symptomatic level allows a preventive regimen to be applied.

Furthermore, as indicated above, these diagnostic methods may also use other target genes or proteins in combinations with SLC6A7, to further increase the reliability, predictability or disease spectrum of the assays or kits.

### DRUG SCREENING

The present invention also provides novel targets and methods for the screening of drug candidates or leads for preventing or treating autism. The methods include binding assays and/or functional assays, and may be performed in vitro, in cell systems, in animals, etc.

It is herein described a method of selecting biologically active compounds, more particularly compounds active on autism said method comprising contacting in vitro a test compound with a SLC6A7 gene or polypeptide according to the present invention and determining the ability of said test compound to bind said SLC6A7 gene or polypeptide. Binding to said gene or polypeptide provides an indication as to the ability of the compound to modulate the activity of said target, and thus to affect a pathway leading to autism in a subject. In a preferred embodiment, the method comprises contacting in vitro a test compound with a SLC6A7 polypeptide or a fragment thereof according to the present invention and determining the ability of said test compound to bind said SLC6A7 polypeptide or fragment. The fragment preferably comprises a binding site of the SLC6A7 polypeptide. Preferably, said SLC6A7 gene or polypeptide or a fragment thereof is an altered or mutated SLC6A7 gene or polypeptide or a fragment thereof comprising the alteration or mutation.

It is also described a method of selecting compounds active on autism said method comprising contacting in vitro a test compound with a SLC6A7 polypeptide according to the present invention or binding site-containing fragment thereof and determining the ability of said test compound to bind said SLC6A7 polypeptide or fragment thereof. Preferably, said SLC6A7 polypeptide or a fragment thereof is an altered or mutated SLC6A7 polypeptide or a fragment thereof comprising the alteration or mutation.

In a further particular embodiment, the method comprises contacting a recombinant host cell expressing a SLC6A7 polypeptide according to the present invention with a test compound, and determining the ability of said test compound to bind said SLC6A7 and to modulate the activity of SLC6A7 polypeptide. Preferably, said SLC6A7 polypeptide or a fragment thereof is an altered or mutated SLC6A7 polypeptide or a fragment thereof comprising the alteration or mutation.

The determination of binding may be performed by various techniques, such as by labelling of the test compound, by competition with a labelled reference ligand, etc.

It is further provided a method of selecting biologically active compounds, more particularly compounds active on autism, said method comprising contacting in vitro a test compound with a SLC6A7 polypeptide according to the present invention and determining the ability of said test compound to modulate the activity of said SLC6A7 polypeptide. Preferably, said SLC6A7 polypeptide or a fragment thereof is an altered or mutated SLC6A7 polypeptide or a fragment thereof comprising the alteration or mutation.

It is also described a method of selecting biologically active compounds, more particularly compounds active on autism said method comprising contacting in vitro a test compound with a SLC6A7 gene according to the present invention and determining the ability of said test compound to modulate the expression of said SLC6A7 gene. Preferably, said SLC6A7 gene or a fragment thereof is an altered or mutated SLC6A7 gene or a fragment thereof comprising the alteration or mutation.

In an other embodiment, it is described a method of screening, selecting or identifying active compounds, particularly compounds active on autism, the method comprising contacting a test compound with a recombinant host cell comprising a reporter construct, said reporter construct comprising a reporter gene under the control of a SLC6A7 gene promoter, and selecting the test compounds that modulate (e.g. stimulate or reduce) expression of the reporter gene. Preferably, said SLC6A7 gene promoter or a fragment thereof is an altered or mutated SLC6A7 gene promoter or a fragment thereof comprising the alteration or mutation. The SLC6A7 gene promoter sequence is disclosed in NM_014228 for instance. Also, SEQ ID NO: 1, up to the first or second ATG codon, comprises a portion of the SLC6A7 gene promoter sequence.

In a particular embodiment of the methods of screening, the modulation is an inhibition. In an other particular embodiment of the methods of screening, the modulation is an activation.

Since the SLC6A7 gene encodes an amino acid transporter, in a particular embodiment, the screening assays comprise a detection or measure of the amino acid transported across a membrane, particularly in neurons, compounds that modulate said transport being selected.

It is further provided a method of selecting biologically active compounds, more particularly compounds active on autism, said method comprising contacting in vitro a test compound with a gene or polypeptide involved in the regulation of the activity of SLC6A7, preferably a CAMK2A gene or polypeptide, and determining the ability of said test compound to bind said gene or polypeptide. Binding to said gene or polypeptide provides an indication as to the ability of the compound to modulate the activity of said target, and thus to affect a pathway leading to autism in a subject. In a preferred embodiment, the method comprises contacting in vitro a test compound with a polypeptide or a fragment thereof involved in the regulation of the activity of SLC6A7, preferably a CAMK2A polypeptide or a fragment thereof, and determining the ability of said test compound to bind said polypeptide or fragment. The fragment preferably comprises a binding site of the polypeptide.

It is further described a method of selecting compounds active on autism, said method comprising contacting in vitro a test compound with a polypeptide involved in the regulation of the activity of SLC6A7, preferably a CAMK2A polypeptide, or binding site-containing fragment thereof and determining the ability of said test compound to bind said polypeptide or fragment thereof.

In a further particular embodiment, the method comprises contacting a recombinant host cell expressing a polypeptide involved in the regulation of the activity of SLC6A7, preferably a CAMK2A polypeptide, with a test compound, and determining the ability of said test compound to bind said polypeptide and to modulate the activity of said polypeptide.

It is also provided a method of selecting biologically active compounds, more particularly compounds active on autism, said method comprising contacting in vitro a test compound with a polypeptide involved in the regulation of the activity of SLC6A7, preferably a CAMK2A polypeptide, and determining the ability of said test compound to modulate the activity of said polypeptide.

It is further described a method of selecting biologically active compounds, more particularly compounds active on autism, said method comprising contacting in vitro a test compound with a gene involved in the regulation of the activity of SLC6A7, preferably a CAMK2A gene, and determining the ability of said test compound to modulate the expression of said gene.

In an other embodiment, it is provided a method of screening, selecting or identifying active compounds, particularly compounds active on autism the method comprising contacting a test compound with a recombinant host cell comprising a reporter construct, said reporter construct comprising a reporter gene under the control of the promoter of a gene involved in the regulation of the activity of SLC6A7, preferably a CAMK2A gene, and selecting the test compounds that modulate (e.g. stimulate or reduce) expression of the reporter gene.

In a particular embodiment of the methods of screening, the modulation is an inhibition. In an other particular embodiment of the methods of screening, the modulation is an activation.

The above screening assays may be performed in any suitable device, such as plates, tubes, dishes, flasks, etc. Typically, the assay is performed in multi-wells plates. Several test compounds can be assayed in parallel. Furthermore, the test compound may be of various origin, nature and composition. It may be any organic or inorganic substance, such as a lipid, peptide, polypeptide, nucleic acid, small molecule, etc., in isolated or in mixture with other substances. The compounds may be all or part of a combinatorial library of products, for instance.

### GENE, VECTORS, RECOMBINANT CELLS AND POLYPEPTIDES

Products for use in diagnosis, therapy or screening are herein described. These products comprise nucleic acid molecules encoding a SLC6A7 polypeptide, vectors comprising the same, recombinant host cells and expressed polypeptides.

More particularly, it is described an altered or mutated SLC6A7 gene or a fragment thereof comprising said alteration or mutation. Nucleic acid molecules encoding an altered or mutated SLC6A7 polypeptide or a fragment thereof comprising said alteration or mutation are also disclosed. Said alteration or mutation modifies the SLC6A7 activity. The modified activity can be increased or decreased. It is further described a vector comprising an altered or mutated SLC6A7 gene or a fragment thereof comprising said alteration or mutation or a nucleic acid molecule encoding an altered or mutated SLC6A7 polypeptide or a fragment thereof comprising said alteration or mutation, recombinant host cells and expressed polypeptides.

It is further disclosed is a vector comprising a nucleic acid encoding a SLC6A7 polypeptide according to the present invention. The vector may be a cloning vector or, more preferably, an expression vector, i.e., a vector comprising regulatory sequences causing expression of a SLC6A7 polypeptide from said vector in a competent host cell.

These vectors can be used to express a SLC6A7 polypeptide in vitro, ex vivo or in vivo, to create transgenic or "Knock Out" non-human animals, to amplify the nucleic acids, to express antisense RNAs, etc.

The vectors typically comprise a SLC6A7 coding sequence according to the present invention "operably linked" to regulatory sequences, e.g., a promoter, a polyA, etc. The term "operably linked" indicates that the coding and regulatory sequences are functionally associated so that the regulatory sequences cause expression (e.g., transcription) of the coding sequences. The vectors may further comprise one or several origins of replication and/or selectable markers. The promoter region may be homologous or heterologous with respect to the coding sequence, and provide for ubiquitous, constitutive, regulated and/or tissue specific expression, in any appropriate host cell, including for in vivo use. Examples of promoters include bacterial promoters (T7, pTAC, Trp promoter, etc.), viral promoters (LTR, TK, CMV-IE, etc.), mammalian gene promoters (albumin, PGK, etc), and the like.

The vector may be a plasmid, a virus, a cosmid, a phage, a BAC, a YAC, etc. Plasmid vectors may be prepared from commercially available vectors such as pBluescript, pUC, pBR, etc. Viral vectors may be produced from baculoviruses, retroviruses, adenoviruses, AAVs, etc., according to recombinant DNA techniques known in the art.

In this regard, it is described a recombinant virus encoding a SLC6A7 polypeptide as defined above. The recombinant virus is preferably replication-defective, even more preferably selected from El- and/or E4-defective adenoviruses. Gag-, pol- and/or env-defective retroviruses and Rep- and/or Cap-defective AAVs. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO95/14785, WO96/22378, US5,882,877, US6,013,516, US4,861,719, US5,278,056 and WO94/19478.

It is further described a recombinant host cell comprising a recombinant SLC6A7 gene or a vector as defined above. Suitable host cells include, without limitation, prokaryotic cells (such as bacteria) and eukaryotic cells (such as yeast cells, mammalian cells, insect cells, plant cells, etc.). Specific examples include E.coli, Kluyveromyces or Saccharomyces yeasts, mammalian cell lines (e.g., Vero cells, CHO cells, 3T3 cells, COS cells, etc.) as well as primary or established mammalian cell cultures (e.g., produced from fibroblasts, embryonic cells, epithelial cells, nervous cells, adipocytes, etc.).

It is also described a method for producing a recombinant host cell expressing a SLC6A7 polypeptide according to the present invention, said method comprising (i) introducing in vitro or ex vivo into a competent host cell a recombinant nucleic acid or a vector as described above, (ii) culturing in vitro or ex vivo the recombinant host cells obtained and (iii), optionally, selecting the cells which express the SLC6A7 polypeptide.

Such recombinant host cells can be used for the production of SLC6A7 polypeptides or of membrane preparations comprising such polypeptides, as well as for screening of active molecules, as described below. Such cells may also be used as a model system to study autism. These cells can be maintained in suitable culture media, such as DMEM, RPMI, HAM, etc., in any appropriate culture device (plate, flask, dish, tube, pouch, etc.).

Further aspects and advantages of the present invention will be disclosed in the following experimental section, which should be regarded as illustrative and not limiting the scope of the present application.

### EXAMPLES

### 1. Identification of an Autism susceptibility locus on human chromosome 5

### A. GenomeHIP platform to identify the chromosome 5 susceptibility gene

The GenomeHIP platform was used to allow rapid identification of an autism susceptibility gene. Briefly, the technology consists of forming pairs from the DNA of related individuals. Each DNA is marked with a specific label allowing its identification. Hybrids are then formed between the two DNAs. A particular process (WO00/53802) is then applied that selects all fragments identical-by-descent (IBD) from the two DNAs in a multi step procedure. The remaining IBD enriched DNA is then scored against a BAC clone derived DNA microarray that allows the positioning of the IBD fraction on a chromosome.
The application of this process over many different families results in a matrix of IBD fractions for each pair from each family. Statistical analyses then calculate the minimal IBD regions that are shared between all families tested. Significant results (p-values) are evidence for linkage of the positive region with the trait of interest (here autism). The linked interval can be delimited by the two most distant clones showing significant p-values.
In the present study, 114 families from the United States (114 independent sib-pairs) concordant for strict autism (as defined by ADI-R) were submitted to the GenomeHIP process.. The resulting IBD enriched DNA fractions were then labeled with Cy5 fluorescent dyes and hybridised against a DNA array consisting of 2263 clones covering the whole human genome with an average spacing of 1.2 Mega base pairs. Non-selected DNA labelled with Cy3 was used to normalize the signal values and compute ratios for each clone. Clustering of the ratio results was then performed to determine the IBD status for each clone and pair.
By applying this procedure the clone FEODBACA28ZH06 (p-value 1.6 x 10⁻⁴) spanning approximately 148 kilo bases in the region on chromosome 5 (bases 149772850 to 149921225) was identified, that showed suggestive evidence for linkage to autism as defined by a p-value of < 7.0 x 10⁻⁴ (Lander and Kruglyak, 1995).

Table 2: Linkage results for chromosome 5 in the region containing the SCL6A7 locus: Indicated is the region corresponding to 1 BAC clone with evidence for linkage plus the flanking region. The start and stop positions of the clones-correspond to their genomic locations based on NCBI Build34 with respect to the start of the chromosome (p-ter).

**Table 2**

| Human chromosome | Clones | Start | End | Proportion of informative pairs | p-value |
|---|---|---|---|---|---|
| 5 | FE0DBACA19ZG04 | 149586232 | 149711662 | 0.78 | 3.60E-01 |
| 5 | FE0DBACA28ZA07 | 149720856 | 149851785 | 0.81 | 1.80E-01 |
| 5 | FE0DBACA28ZH06 | 149772850 | 149921225 | 0.92 | 1.60E-04 |
| 5 | FE0DBACA28ZC08 | 149773035 | 149773128 | 0.76 | 4.80E-01 |
| 5 | FE0DBACA16ZA06 | 150001931 | 150170529 | 0.84 | 2.40E-02 |

### B. Identification of an autism susceptibility gene on chromosome 5

By screening the aforementioned 148 kilo bases in the linked chromosomal region plus 200 kb of the region flanking the positive clone, we identified the solute carrier family 6 (neurotransmitter transporter, L-proline), member 7 (SLC6A7) gene and the calcium/calmodulin-dependent protein kinase (CaM kinase) II alpha (CAMK2A) gene as candidates for autism and related phenotypes. These genes are indeed present in the flanking interval, with evidence for linkage delimited by the clone outlined above.

SLC6A7 gene encodes a predicted 636-amino acid polypeptide (mRNA 1.9 kb) and spreads over 21.9 kb of genomic sequence. The protein encoded by the gene is a member of the gamma-aminobutyric acid (GABA) neurotransmitter gene family which includes Na- and C1-dependent plasma membrane carriers for neurotransmitters, osmolites, metabolites and the amino acid proline. It contains a sodium:neurotransmitter symporter domain. The transporter can directly terminate the action of proline by its high affinity sodium-dependent reuptake into presynaptic terminals. L-proline is a putative synaptic regulatory molecule in the central nervous system that is synthesized from ornithine in synaptosomes.

Most importantly this transporter is selectively localized to a subset of presynaptic axon terminals, forming asymmetric excitatory-type synapses typical of glutamatergic synapses (Renick et al., 1999).

Furthermore, recent findings indicate that SLC6A7 contributes to the molecular heterogeneity of glutamatergic terminals and suggest a novel presynaptic regulatory role for SLC6A7 in excitatory transmission at specific glutamatergic synapses (Crump et al., 1999).

The search for specific L-proline uptake inhibitors to study the physiological role of SLC6A7 has lead to the identification of enkephalins that competitively inhibited high affinity L-proline uptake through a direct interaction with the L-proline transporter protein. Leu- and Met-enkephalin and their des-tyrosyl derivatives, eg des-tyrosyl-Leu-enkephalin, potently and selectively inhibited L-proline uptake in rat hippocampal synaptosomes and in SLC6A7-transfected HeLa cells (Fremeau et al., 1996). Galli et al. (1999) have shown that SLC6A7 is electrogenic, and that L-proline, L-pipecolate (PIP), L-norleucine and sarcosine are substrates of SLC6A7. PIP is either a substrate for the transporter or a rare antagonist of neurotransmitter uptake.

Jayanthi et al. (2000) examined the role of [Ca2+]I and Ca(2+)-dependent kinases in the modulation of SLC6A7. They showed that beta-PMA (phorbol 12-myristate 13-acetate), an activator of protein kinase C (PKC), inhibits L-proline uptake. Down-regulation of PKC by chronic treatment with beta-PMA enhances SLC6A7 function indicating SLC6A7 regulation by tonic activity of PKC. Thapsigargin, which increases [Ca2+]I levels by inhibiting Ca(2+)-ATPase, inhibits SLC6A7 and exhibits additive inhibition when co-treated with beta-PMA. A Ca2+/calmodulin-dependent kinase II (CAMK2) inhibitor, but not BIM (a PKC inhibitor) prevents the inhibition by thapsigargin. These data suggest that PKC and CAMK2 modulate SLC6A7 and that thapsigargin mediates its effect via CAMK2. It appears that Ca2+ is differentially regulating SLC6A7. Initially, Ca2+ enhances proline transport but eventually inhibits transport function through the CAMK2 pathway.

The CAMK2A gene encodes two isoforms with a predicted 489-amino acid polypeptide (mRNA 4836 bp, cDNA 1470 bp) for isoform 1 and a predicted 478-amino acid polypeptide (mRNA 4803 bp, cDNA 1437 bp) for isoform 2. The CAMK2A gene spreads over 70.3 kb of genomic sequence. The proteins encoded by this gene are members of the serine/threonine protein kinases family, and the Ca(2+)/calmodulin-dependent protein kinases subfamily. Ca(2+)/calmodulin-dependent protein kinase 2 (CAMK2) comprises a family of different isoforms that are derived from four genes (alpha, beta, gamma and delta). The alpha and beta subunits are the predominant isoforms in the brain, where they form dodecameric holoenzymes that are composed of either one or both types of subunits. CAMK2 is enriched at synapses and is the main protein of the postsynaptic density (PSD). CAMK2 is central to the regulation of glutamatergic synapses. The alpha chain encoded by the CAMK2A gene is required for hippocampal long-term potentiation (LTP), an activity-dependent strengthening of snynapses that is thought to underline some forms of learning and memory. Most importantly this kinase translocates to synapses, where it binds directly to the NMDA (N-methyl-D-aspartate) receptor, a subtype glutamate receptor, within the PSD. Binding results in phosphorylation of the NMDA receptor. The translocation appears to be induced by glutamate (Bayer et al., 2001). CAMK2 binds to at least two other proteins in the PSD, densin-180 and a-actinin 4 (Strack et al., 2000; Walikonis et al., 2001).

CAMK2 can be activated to different degrees, with decay times that depend on the magnitude of the Ca2+ signal and the properties of the phosphatases that dephosphorylate the kinase. In the cytosplasm, CAMK2 is dephosphorylated primarily by protein phosphatase 2A (PP2A), whereas in the PSD, the kinase is almost exclusively dephosphorylated by protein phosphatase 1 (PP1) (Strack et al., 1997). The ability to dephosphorylate CAMK2 seems to depend on the fact that PP1 is immobilized in the PSD by scaffold proteins that include spinophilin, neurabin, yotiao and intermediate filaments (Watanabe et al., 2001).

There is strong evidence that LTP involves a postsynaptic process, which selectively enhances AMPA (a-amino-3-hydroxy-5-methyl-4-isoxazole propionic acid)-receptor mediated transmission (Malenka and Nicoll, 1999). CAMK2 phosphorylates AMPA receptors that are already localized at the synapses, enhancing their conductance. The AMPA receptor is an other glutamate subtype receptor.

CAMK2A might be directly responsible for the persistence of LTP and therefore have a learning and memory function. Silva et al. (1992a, 1992b) showed that mice with a mutation in the Camk2a gene were deficient in LTP and hippocampus-dependent spatial learning tasks indicating that CAMK2A is involved in learning and memory. These mice also suffered epileptic seizures.

Mice heterozygous for a null mutation of Camk2a show normal hippocampal LTP, but no cortical LTP. In behavioral tasks, these animals learn normally, but subsequently forget, presumably because of the normal tranfer of information from hippocampus to cortex is not possible (Frankland et al. (2001).

Autophosphorylation at the threonine-286 residue endows CAMIC2A with the ability to switch from a calmodulin-dependent to a calmodulin-independent state. Giese et al. (1998) introduced a thr286-to-ala mutation that blocked autophosphorylation at thr286 of CAMK2A. This mutation resulted in a kinase that was unable to switch to its calmodulin-independent state, but did not affect calmodulin-dependent activity. Eliminating Thr286 posphorylation not only blocks LTP, but also interferes with experience-dependent plasticity in vivo. Behavioral tests show that memory is strongly impaired by this mutation. Thus, CAMK2A is involved in basic synaptic processes that store behaviorally relevant information.

In addition, it has been shown that mice deficient in the Camk2a gene showed behavioral abnormalities (Chen et al., 1994). The heterozygous mice exhibited a well-circumscribed syndrome consisting primarily of a decreased fear response and an increase in defensive aggression, in the absence of any measured cognitive deficits.

The search for specific inhibitors to test whether CAMK2 is required for LTP induction has lead to the identification of specific inhibitors of this kinase (Malinow et al., 1989). Peptides modeled after the autoinhibitory region (for example, autocamtide-2 or a peptide comprising residues 273-302 of CAMK2) block the Ca2+-independent activity of the enzyme without interfering with other calmodulin-dependent processes (Lisman et al., 2002). Introduction of autocamtide-3 derived peptide inhibitor (AC3-I) into the postsynaptic cell completely blocks the induction of LTP producted by pairing but does not affect LTP maintenance (Otmakhov et al., 1997). Membrane-permeant inhibitors of CAMK2, such as KN62 and KN93, block the Ca2+-dependent activity of the enzyme by interfering with calmodulin binding, and prevent LTP induction by brief titanic stimulation, a standard LTP-inducing protocol (Otmakhov et al., 1997).

Jayanthi et al. (2000) examined the role of [Ca2+]I and Ca(2+)-dependent kinases in the modulation of SLC6A7, a L-proline neurotransmitter transporter. It appears that Ca2+ is differentially regulating SLC6A7. Initially, Ca2+ enhances proline transport but eventually inhibits transport function through the CAMK2 pathway.

CAMK2 has been implicated in the action of anticonvulsants, benzodiazepines, and antipressants. Recently, Celano et al. (2003) showed that CAMK2 also plays a role in the action of different drugs employed for the treatment of psychiatric diseases.

It has been hypothesized that the severe disruptions observed in autism may be linked to GABAergic inhibition, resulting in excessive stimulation of glutamate specialized neurons and loss of sensory gating (Hussman, 2001).

In a hypoglutamatergic rodent model, certain behaviors that might have relevance for the cognitive impairments seen in autism were observed (Nilsson et al., 2001).

Reductions in glutamic acid decarboxylase 65 and 67 kDa levels may account for reported increases of glutamate in blood and platelets of autistic subjects (Fatemi et al., 2002). Glutamic acid decarboxylase deficiency may be due to or associated with abnormalities in levels of glutamate/gamma amino butyric acid, or transporter/receptor density in autistic brain. Furthermore, a decrease of glutamate receptor density has been observed in the cerebellum of autistic patients (Purcell et al., 2001).

Taken together, the linkage results provided in the present application, identifying the human SLC6A7 gene in the immediate neighbourhood of the interval of genetic alterations linked to autism on chromosome 5, with its regulatory role at glutamatergic synapses, we conclude that alterations (e.g., mutations and/or polymorphisms) in the SLC6A7 gene or its regulatory sequences may contribute to the development of human autism and represent a novel target for diagnosis or therapeutic intervention.

### 3. Association study

The same families that have been used for the linkage study were also used to test for association between a specific phenotype (here autism) in question and the genetic marker allele or haplotypes containing a specific marker allele using the transmission disequilibrium test (TDT). The TDT is a powerful association test as it is insensitive to population stratification problems in the tested sample. Briefly, the segregation of alleles from heterozygous parents to their affected offspring is tested. The portion of alleles transmitted to the affected offspring compared to the non-transmitted alleles is compared to the ratio expected under random distribution. A significant excess of allele transmission over the expected value is evidence for an association of the respective allele or haplotype with the studied autism phenotype.

The results of this analysis show that certain alleles of the SLC6A7 gene are positively associated with autism and therefore increase the susceptibility to disease. In the tested population, the allele G of SNP5, the allele A of SNP6, the allele T of SNP7, the allele C of SNP8 and the allele G of SNP10 are correlated with autism as determined by TDT (p-values ranging from 0.03 to 0.006). In contrast, the opposite alleles of these SNPs are under-transmitted to autistic individuals showing that these alleles help protect from the disease.

The example of the transmission to autists of the alleles of SNP5 to SNP10 is given in Table 3.

**Table 3**

| SNP | Allele | Transmitted to autists | Not transmitted to autists | p- value |
|---|---|---|---|---|
| SNP5 | C | 59 | 85 | 0.03 |
| SNP5 | G | 85 | 59 | 0.03 |
| SNP6 | A | 86 | 60 | 0.03 |
| SNP6 | G | 60 | 86 | 0.03 |
| SNP7 | C | 53 | 81 | 0.016 |
| SNP7 | T | 81 | 53 | 0.016 |
| SNP8 | C | 85 | 53 | 0.006 |
| SNP8 | G | 53 | 85 | 0.006 |
| SNP10 | A | 59 | 88 | 0.017 |
| SNP10 | G | 88 | 59 | 0.017 |

In addition, haplotypes were constructed for SNP3, SNP4, SNP5, SNP6, SNP7, SNP8, SNP9, SNP10, SNP12, and SNP14 to identify the phase for all SNPs.

The results of this analysis in the tested population showed that certain haplotypes, all characterized by the presence of allele C of SNP8 are strongly associated with autism, while certain haplotypes devoid of allele G at SNP8 are preferentially not transmitted to autists. Examples are the haplotypes G-T-C for SNP4-SNP7-SNP8, p = 0.009741 and haplotype C-C-G for SNP8-SNP9-SNP14, p = 0.00235. Haplotypes that carry allele C instead of allele G at SNP8 show evidence to be underrepresented in autistic subjects. An example is the haplotype C-G-G for SNPS-SNP6-SNPB, p = 0.02742.

Examples of haplotypes with preferential transmission and non-transmission of SNP8 to autists are given in Tables 4 and 5

**Table 4**

| SNPs used to construct haplotype | Haplotype | Frequency of haplotype transmitted to autists | Frequency of haplotype not transmitted to autists | p- value |
|---|---|---|---|---|
| SNP3-SNP5.SNP8 | T-G-C | 0.3838 | 0.2629 | 0.0157 |
| SNP4-SNP5-SNP8 | G-G-C | 0.3865 | 0.2632 | 0.01226 |
| SNP5-SNP6-SNP8 | C-G-G | 0.4615 | 0.5769 | 0.02742 |
| SNP5-SNP6-SNP8 | G-A-C | 0.522 | 0.4066 | 0.02714 |
| SNP5-SNP7-SNP8 | C-C-G | 0.4775 | 0.5899 | 0.03341 |
| SNP5-SNP7-SNP8 | G-T-C | 0.5056 | 0.3933 | 0.0329 |
| SNP5-SNP8-SNP9 | G-C-C | 0.4311 | 0.3247 | 0.0433 |
| SNP5-SNP8-SNP10 | C-G-A | 0.4739 | 0.582 | 0.04201 |
| SNP5-SNP8-SNP12 | G-C-A | 0.4066 | 0.2847 | 0.01758 |
| SNP2-SNP7-SNP8 | G-T-C | 0.2243 | 0.1395 | 0.05133 |
| SNP3-SNP7-SNP8 | T-T-C | 0.38 | 0.2551 | 0.01382 |
| SNP4-SNP7-SNP8 | G-T-C | 0.3838 | 0.2548 | 0.00974 |
| SNP5-SNP7-SNP8 | C-C-G | 0.4775 | 0.5899 | 0.03341 |
| SNP5-SNP7-SNP8 | G-T-C | 0.5056 | 0.3933 | 0.0329 |
| SNP6-SNP7-SNP8 | A-T-C | 0.523 | 0.408 | 0.03141 |
| SNP6-SNP7-SNP8 | G-C-G | 0.4598 | 0.5747 | 0.03172 |
| SNP7-SNP8-SNP9 | T-C-C | 0.4433 | 0.3306 | 0.038 |
| SNP7-SNP8-SNP10 | C-G-A | 0.4792 | 0.5858 | 0.04927 |
| SNP7-SNP8-SNP12 | T-C-A | 0.3991 | 0.2848 | 0.02686 |

**Table 5**

| SNPs used to construct haplotype | Haplotype | Z score | Frequency of transmitted haplotype in the tested population | p- value |
|---|---|---|---|---|
| SNPS-SNP8-SNP14 | G-C-G | 2.836 | 0.1066 | 0.00456 |
| SNP6-SNP8-SNP14 | A-C-G | 2.893 | 0.1095 | 0.00381 |
| SNP7-SNP8-SNP14 | T-C-G | 2.808 | 0.1049 | 0.00498 |
| SNP8-SNP9-SNP14 | C-C-G | 3.0411 | 0.1269 | 0.00235 |

### 4. Identification of nucleotide changes

96 unrelated affected individuals were included in the mutation screen. Primers were designed to amplify the coding region of the SCL6A7 gene. Each primer contained a tail comprising of M13F and M13R sequences that are marked in capital letters, respectively, to facilitate direct sequencing of the PCR products using the M 13 primers. The sequences of the primers are provided below:

| | | |
|---|---|---|
| Pro-01-Ft | TGTAAAACGACGGCCAGTagtgttccttgcccaactgt | SEQ ID NO: 13 |
| Pro-01-Rt | CAGGAAACAGCTATGACCctctccaaccctcctccag | SEQ ID NO: 14 |
| Pro-02-Ft | TGTAAAACGACGGCCAGTctggcctcagtcttctccc | SEQ ID NO: 15 |
| Pro-02-Rt | CAGGAAACAGCTATGACCgccttggcccatcac | SEQ ID NO: 16 |
| Pro-03-Ft | TGTAAAACGACGGCCAGTctagagctgggcttttggg | SEQ ID NO: 17 |
| Pro-03-Rt | CAGGAAACAGCTATGACCcctcagcagtgcagggtc | SEQ ID NO: 18 |
| Pro-04-Ft | TGTAAAACGACGGCCAGTtpctccatgtctgtggagc | SEQ ID NO: 19 |
| Pro-04 Rt | CAGGAAACAGCTATGACCgccttctccaggaagcct | SEQ ID NO: 20 |
| Pro-05-Ft | TGTAAAACGACGGCCAGTtggcactgagtcaaggtcc | SEQ ID NO: 21 |
| Pro-05-Rt | CAGGAAACAGCTATGACCctctttccactctccagctca | SEQ ID NO: 22 |
| Pro-06-Ft | TGTAAAACGACGGCCAGTtgccttccttctctgtcctt | SEQ ID NO: 23 |
| Pro-06-Rt | CAGGAAACAGCTATGACCctacccaacacacatgctca | SEQ ID NO: 24 |
| Pro-07-Ft | TGTAAAACGACGGCCAGTtctgagtgtgcgtatgggag | SEQ ID NO: 25 |
| Pro-07 Rt | CAGGAAACAGCTATGACCgccagagatctcgttgcag | SEQ ID NO: 26 |
| Pro-08-Ft | TGTAAAACGACGGCCAGTgtagcagagaacgaggccc | SEQ ID NO: 27 |
| Pro-08-Rt | CAGGAAACAGCTATGACCgactcccgctttcaattctg | SEQ ID NO: 28 |
| Pro-09-Ft | TGTAAAACGACGGCCAGTcgggccttaagcagtttaga | SEQ ID NO: 29 |
| Pro-09-Rt | CAGGAAACAGCTATGACCggtcaggaaggctgagagtg | SEQ ID NO: 30 |
| Pro-10-Ft | TGTAAAACGACGGCCAGTcgctgcctgtttcctgtt | SEQ ID NO: 31 |
| Pro-10-Rt | CAGGAAACAGCTATGACCaagaagggactgtgaggtcc | SEQ ID NO: 32 |
| Pro-11-Ft | TGTAAAACGACGGCCAGTggcctagatagccaggtgagt | SEQ ID NO: 33 |
| Pro-11-Rt | CAGGAAACAGCTATGACCgccagagatctcgttgcag | SEQ ID NO: 34 |
| Pro-12-Ft | TGTAAAACGACGGCCAGTgctgttagtgttccttgccc | SEQ ID NO: 35 |
| Pro-12-Rt | CAGGAAACAGCTATGACCGAATGCCTTGTCTGTCCCTG | SEQ ID NO: 36 |
| Pro-13-Ft | TGTAAAACGACGGCCAGTTCTGTCTGGGTGTCTATGCG | SEQ ID NO: 37 |
| Pro-13-Rt | CAGGAAACAGCTATGACCgggtgcatcctcagacctt | SEQ ID NO: 38 |
| Pro-14-Ft | TGTAAAACGACGGCCAGTatctgcaggccaggggag | SEQ ID NO: 39 |
| Pro-14-Rt | CAGGAAACAGCTATGACCgcagctatctgggcttcact | SEQ ID NO: 40 |
| Pro-15-Ft | TGTAAAACGACGGCCAGTagctccccagaagccact | SEQ ID NO: 41 |
| Pro-15-Rt | CAGGAAACAGCTATGACCtacatgctgagactgtgggg | SEQ ID NO: 42 |
| Pro-16-Ft | TGTAAAACGACGGCCAGTtgagtgtaagtggccgtgtg | SEQ ID NO: 43 |
| Pro-16-Rt | CAGGAAACAGCTATGACCcctgctgccacagacgag | SEQ ID NO: 44 |
| Pro-17-Ft | TGTAAAACGACGGCCAGTgatagaattctgacccccagc | SEQ ID NO: 45 |
| Pro-17-Rt | CAGGAAACAGCTATGACCcagagatctcgttgcaggc | SEQ ID NO: 46 |
| Pro-18-Ft | TGTAAAACGACGGCCAGTcacttcttgccaggagaagg | SEQ ID NO: 47 |
| Pro-18-Rt | CAGGAAACAGCTATGACCgctccccagggtagactcc | SEQ ID NO: 48 |
| Pro-19-Ft | TGTAAAACGACGGCCAGTcccagtacctgggtccct | SEQ ID NO: 49 |
| Pro-19-Rt | CAGGAAACAGCTATGACCgactcccgctttcaattctg | SEQ ID NO: 50 |

The resulting amplification products were directly sequenced in both directions using dye-terminator sequencing chemistry to identify rare nucleotide changes (mutations) and polymorphisms (allele frequency >1%) in the gene.

A total of 12 nucleotide changes were detected in the coding region of the gene plus the flanking intron regions in close proximity of the splice sites (for positions see table 6). Three of these resulted in changes of the amino-acids in the respective codons, as illustrated in table 6.
Furthermore, an additional ATG (start codon, position 229 in SEQ ID NO: 1) has also been identified upstream, in frame with the ATG used as a reference for the start of the coding region in the public databases (located at position 472 in SEQ ID NO: 1). This alternative ATG would result in a slightly longer protein. Positions for the variants are therefore given for the two alternative proteins whereby the position based on the reference sequence of Seq No. ID 2 is listed first.

**Table 6 :**

| ID | Location in gene | Nucleotide position in Seq No ID 1 | Nucleotide change | Variation and position in Seq No ID 2 or extended protein shown below | Frequency |
|---|---|---|---|---|---|
| Mut1 | exon 1 | 289 | A/G | 5'UTR variation or I21V | 48% |
| Mut2 | intron 2-3, -5 bp from 3' splice site | 6,854 | C/T | splice site variation | 48% |
| Mut3 | exon 5 | 9,477 | C/T | L230L or L311L | 0.5% |
| Mut4 | exon 8 | 12,772 | G/A | G338S or G419S | 0.5% |
| Mut5 | exon 9 | 13,881 | T/C | F386F or F467F | 31% |
| Mut6 | exon 9 | 13,917 | T/C | D398D or D479D | 29% |
| Mut8 | intron 11-12, -3 bp from 5' splice site | 14,778 | G/A | splice site variation | 19% |
| Mut9 | intron 12-13, -16 bp from | 15,087 | G/A | S582S or S663S | 4% |
| Mut10 | exon 14 | 19,607 | C/T | R587T or R668T | 0.5% |
| Mut11 | exon 14 | 19,707 | C/T | T620M or T701M | 0.5% |
| Mut12 | exon 14, +2 bp after stop codon | 19,761 | G/A | 3' UTR variant | 0.5% |

Mut1 in exon 1 is identical with SNP6 that has been used in the association study. SNP6 was independently associated with autism and was also part of the haplotypes SNP5-SNP6-SNP8, SNP6-SNP7-SNP8 and SNP6-SNP8-SNP14 that have been transmitted more often to autistic patients than expected by chance.

Mut2 and Mut8 occurred close to the splicing sites and could therefore affect the splicing at these sites.
The mutation C to T in codon 338 occured in the 7th transmembrane domain and lead to a non-synonymous amino acid change from glycine to serine. The mutations in codon 587 and 620 occured in the cytoplasmic domain and lead to a non-synonymous amino acid change from arginine to threonine and threonine to methionine, respectively. All of these mutations represent valuable targets for screening or diagnosis purposes, as disclosed in the present application. Furthermore, these mutations, and the corresponding polypeptide sequence represent valuable epitopes to generate specific antibodies, Moreover, a particular object of this invention is a SLCA6A7 polypeptide comprising an extended N-terminal amino acid sequence as follows:
MRAQQCTLPQ PRALRRDRQG IRSALPALHA RSRQTAAPAS VPAPAGAREP RGQRRSGQRT ISRALALCAP GQLSPGHPLS K (SEQ ID NO: 51)

This sequence results from the use of an upstream ATG start codon, as discovered by the inventors. The resulting full length SLCA6A7 polypeptide comprises 717 amino acid residues. Also, a polypeptide comprising all or part of SEQ ID NO: 51 represents a particular object of the present invention. Such a polypeptide more preferably comprises at least 5 consecutive amino acid residues of SEQ ID NO: 51, even more preferably at least 7, 8, 9, 10, 12, 15 or 20. The polypeptide typically comprises one epitope.

### INFORMATION FOR SEQUENCES 1 TO 12

SEQ ID NO: 1 : Sequence of the SCL6A7 gene (genomic DNA) used as reference to position mutations 
GCACAGCAAAGACGACGCAGGGGGTGGCTGTTAGTGTTCCTTGCCCAA
CTGTGGAAAGGGAGTCCCAGAGAGGGCAGGTCGCTTGCCCAAGGTCACGC
AGAAAGCCGAGGGTTTCCGTCGCGAACCCCCCTCCCCACTCGCTCAGGGC
TCTCCAATCCGCAGCTCTGCGTGCGGGGGCGCGCGCATCCCCCCGCCGTC
CGTCCGTCAGCTGTCTGTCTGGGTGTCTATGCGGGCGCAGCAGTGCACCC
TTCCCCAGCCTCGGGCGCTGCGCAGGGACAGACAAGGCATTCGCAGCGCC
CTGCCCGCGCTCCACGCCCGCAGCCGCCAGACGGCAGCGCCTGCGTCCGT
GCCCGCCCCAGCCGGTGCGCGGAGCCGCGGGGGCAAAGGCGCAGTGGCC
AGCGGACCATCTCTCGTGCCCTCGCTCTCTGCGCTCCGGGCAGCTGAGC
CCCGGCCACCCGCTCTCCAAGATGAAGAAGCTCCAGGGAGCTCACCTCCG
CAAGGTAGGGCACGAGGGCGGGGGCGCTGGGGGTGCACCTGGAGGAGGGT
TGGAGAGACCCGCCCCCAACGAGGCCCCTGGGAAGGTCTGAGGATGCAC
CCAGACCAGCTTCGGGCTCTGGGGAAGGCCCGACTCAGCTGGATAACAGG
GAGGGTCAGGGTCACGGTCTGCGCCCCCACCTCTGCCCCTGCCATCTGGG
GTTCGGGATGTAGTATGAGGGGAGTCCTGGTTCACTGGGCCAGGCCTATG
AACAGGTGTCTGCAGTCCCCGGAGGCGCGGGGTAGGGGCGGCCGGGGCCA
GGCACCCACCTCTTCCCCAATTCCACCCTGCTGCTCCCCGCCAGGAGCTG
ATTGCCGGGTGTGGGGGTATTGGAATACCTGAGCGTTGAGCTGGACTCA
TCTGAGGGGTGGGGAGTGGAGGCGGTCATCCATACTGAAGCCGGCTCCC
TGAGCCTGCGGGAAGACTCTCCTCTTTCCTGCCTGCTCCCTCCCCGCCCC
CTTAGCTTGCCTGCTGAGACCCCAGGCTGCCCCTCAGCAGGGCTGAAGGG
AGGCAAAGACAGGGAGGGGGCTATCGGAGGCAGGAGGATATGATCAATGA
AGATGGAAGCTGGTATGGAGAGTGGCTGTGGGCCCAGACCTCAGGCTCT
CCCTACCTTGCCTTGTGGGATTGGACCTCTCAGCCAAGCTGTCAGGATAT
GGGGAGGGGGAATACTGGGGGACGTTTTCTGGCTTGTACCAGTCCTAACT
AAGGAGTCAGATCTCCTGAATACTATTTCTGCCGCTGCCACTTGCCTGGT
GACTTTGGACAAGTTCATTCTCTAACCTGAACCTCCTCTGGCTAAGCCCT
AGCCTGGAGGCACCAAAACAGGCCCCACTGGGTGTGTGAGGTGTGGGGAA
GAATGCTAAAGGGCTGGTAGATGTGAGAAGACTGTTCTCAGGGGCTGGTG
TTATCAACCTCCCTACATACACACACATTCACACTCACACTCACTCACAC
ACACACTCATTCATTCAAGTTCTCTGCTCTGGGGCAGCTGGGCTTGGAAC
CACTGTGGTGTGTCTTTTTCTTTTTCTTGTTTTTTTTTTTGAGACGGA
GTCTCGCTCTGTCACCCAGGTTGGAGTGCAGTGGTGTGATCTCGGCTCAC
TGCAAGCTCTGCCTCCCGGGTTCACGCCATTCTCCTGCCTCAGCCTCCCG
AGTAGCTGGGACTATAGGCGCCCGCCACCACGCCTGGCTAATGTTTTGTA
TTTTTAGTAGAGACGGGGTTTCACCGTGTTAGCCAGGATGGTCTTGATCT
TCTGACCTCGTGATCCGCCCGCCTCGGCCTCCCAAAGTGCTGGGATTACA
GGTGTGAGTCACTGCGCCCAGCTGGTATGTCTTTTTATTCACTGACATG
AGTGACTTCTTGAGACCATTTGTGAGTCTCTTGGGTCCTGTTTCCATTCT
TTTCCACTCTCCTTCTCTTCCCTCTCTGCCCTCCCTCCCCACAAGAGGCC
CTGTCCCTCTTGCTGGGTGCTGGGCTGGGGGGTTAGCAAGCTTTATCTCA
TTTAATCCTTTAACACTTCCAGGGGCTAGGTCTTAGTAGCCCCACTTTAT
AAATAAGGCAACTGAGGCACAGAGGGGTGAGACTCACTGCTTACCTGCTG
GCAGGCTAGGCAACCTAGAGCCAACAGGGCAGAGGGTTGGTTCTAGGTTG
CTACCTCCTCTCAGGGTCCTTTCCCTTTTCTCAAGCAGAATTGGGCGGGA
ATGAGAAAATGCCAGATTTCCGCAAAGGAGCAGCATTGCTCAGAGATGAA
CACTGGACTTAGAGTCAGGGGATCTAGGTCCAAGTCTTGCTGTGCCTTTA
ACTTGCTGTTTGACCTTGGGCTCATCCCTTCTTCCTGAAACTTGGTTTCC
TAATCTGCACAATGATGAAGCTGGACCCCAGATTCCTATTGTCTCTCTCT
GTCCTGAAGAGTCTTGGCTTGGGCAGTCTTGAGTCTCCCAGCCCCTGCTG
TGAGTCCCTAAAGGCGAGCATGCAGGTCTGACAAACTCAGCTGTGTGTTC
CTGCATGCCCCTGGGTAGGGAGTGGAGATTCAGGAGTGGGAGGCTTAC
ACCCAGGAAGAAGTATGAAGCAACTCATTCAGCTTCCAGCCCTGCCCCAG
CTATTTGATTGCTTTCTTCCATCTCCCAACCAGCCCCATCCTCACTGTGC
 
CAGCCTCCTGGGATTCACCCCATGGTGGGGTTGGAACTCCAGTTGGGCT
CCTGATTGGCTCAGCTTTGGAGGACCCTATCTCCTGCCCCTGGAGCAGAA
GCAGGCTTCCCTCTGATTGGGGAGGAGGGGGACGAGCTGGCAGCATCCT
GCTCTTGGAACTCTAGTCACCATGGAAACAGAGAACCGGGACCGCTCCAT
GAGCATGGAAAGGGCAGCTTCTCTGGAGGGATCAGAGGATCCTCATTACT
GGGACCTCCTGCTAACTGGCCTGAATGGTGCTGGGATGGCGGGGGGTGT
CTCAGGGTAACAGGCCTGCTCACCACCCAACACCAGGAACAGTGGCAACC
ATTCCTCCCGGAGCTCACCTGGGGTGAGAGGTTGCTGAAGGCTGATGCTC
ACCCAGCTGGAGAGGCAGCCTCAGGGAACGGGGCATTGGAGGGGAAGGGT
GGGTTTCACTTAAGTCTGGGATCTGCTGCTCCTGTCACCTCAGCATGTCC
ATGATCACAGCTAAAGAACTTCCAGGCATGTATTTGTTTCACCCCTTGCA
GCCTAGTTACTTTAAAAACAGCTCTTCATGCTCTGTCATTGTCATCGTCA
CCCATAATAATGTCTGGCTGTTCCTGTCACCACGGCCCCTGTGGCCAATA
GCCAGTTTCTGGCAAAATCACCACTGTGGTCACCATCACTGAGGGTCTCC
TAAGGGAGAATGCCCCATGGACCTCTCACCCACTTTGAAGCATGAAAGGA
GACCTTAGGAACAGCTGGCCTCCGCTCCCCTCTGGGCATGCATCTCCTCT
CCAGGGACAGCAAGATTATGACTTTCTGACACAGATCACGGCTTAAGTAC
CTTCTTCCTGCTCATGAGCCAAAATTCACAAAATTCACCCAGCCCTCAGG
CTCTCCCTACCTTGCCTGGTGGGGTTTGACCCCCCCAGCCAAGCTGTCAG
CATGCGGGGAGGGGGAGTATTGGGGAAGTTTTCTGGCTTGTGACTGAAAA
GTACCAGTCCTAACCAAGGAGTCAGATCTCCTTGGTTCTTAGTAGGCCCC
ATTTGAGCCACGGAGCATCAGTGGATTCCCTCCCTCAGGGGAACCCCCAG
GTCCCTCCAAGTCCTCTCCTTTCTATGCTGAGGGCTCCCAGCTTCTTTCT
GTTGTCCTCCTATCACCTCTTTTCCTGAACACCCTCCAGCCTGGCCACCT
GCCCTTTGGACATGCTCCATATTGTCTGCATCTTTCTTAAAATATGGTGC
CCGGAACGGAGGAACAAAACTCCAGCACTGGTGGGTGAAGGAGACCTGGC
TTTCGCATCAGGAAGCCTTGGGCTCCAGACCTAGTTCTTCCACTCACCAA
CCTGGTAGAAACCTCGGGTATGTCATGACACCTCTTTGAGCCTCAGTTTT
CCTCATGACGAAAATGGAGCTTAGATGAATACTGAGCTCAAAAAGTTGTT
CTGAGAATGAAATCAGGTAATGTGAGTGAGATGTCTAGTGCAGTGGGCAC
ATAGTAAGTGCTCAATATACAGTAACTTACTAAGATCATCATCATGCTCT
TCATCTTCGTCATCCTCATCCTCATCAAAATAGGACCATCCAGCCGGCCA
CGGTGGCTCATGCCTGTAATCCCAGCACTTTGGGAGGCCGAGGCGGGTGG
ATCACGAGGTCAGGAGACCAAGACCATCCTGGCTAACACGGTGAAACCCT
GTCTCTACTAAAAATACAAAACATTAGCCGGGCGTGGTGGTGGGCGCCTG
TAGTCCCAGCTACTCGGGAGGCTGAGACGGGAGAATTGCTTGAACCCGGG
AGGTGGAGGTTGCGGTGTGCTGAGGTTGTACCACTGCACTCCAGGCTGGG
CAACAGAGGGAGACTCTTTCTCAATTAAAAAAAAAAAAAAAAAAGGACTAT
CCCTTGATTTGGCATCTGTGTTAGCCAGACTAAGGTCAGGGTAGCTTTGT
TAGTGCCTAAGCTACCCTGCTGGCCCAGTAAGTTTTGGTCGCCTAAAATC
CTGAGGCTCTCCTGCATATGACAAGGTTAGTCCAGTTGAGAGCACTTGAT
TATCTGGACTGAGAATGTCTTTTCATTTAGCCCTGTTCAATTCCAGAGTC
TTGTCTTGAGGTCACATTTCTGGCCTGGCCTCAGTCTTCTCCCCAACCTC
TTTGGTCTCTCTCATTGGCAGCCTGTCACCCCAGACCTGCTGATGACCCC
CAGTGACCAGGGCGATGTCGACCTGGATGTGGACTTTGCTGCACACCGGG
GGAACTGGACAGGCAAGCTGGACTTCCTGCTGTCCTGCATTGGCTACTGT
GTAGGCCTGGGGAATGTCTGGCGCTTCCCCTATCGAGCGTACACCAATGG
AGGAGGTATGGGCCTGAGGTCCTGTCGGAGGGTGTCTGGGGTGATGGGCC
AAGGCCCTGGGGTACAGTGAGCTTTTAGGTGGCCTCAGCCACTGTCTCCT
AGGCGACCTTGGGCAGAACTCATTGAAAGTGGTTTATAGTAACTTGGGGC
TGGGAGGAAGGAGTTTACCCTGTCTTGTCCTTCTCATCACCCAAGCCCTT
CCTTCCTCCCTCTCATCCCCAGGAAGGATACCCACATGGTTCCCTCTCTA
AGGCTGGCCACCCCACGCCCTGCACGGTGGGCAGAGGGAGCTGCCTCTGC
TTCGTGATAAGGAGCTGTATCTGCCTGCCGATGATCACTGCACTTTCAGT
GTTCTCCCCACCCCCTCCTTCCTCCACCTCCTCCTGAGCTGTGCTCCATC
AATCATTCCCTCTCTTGAGTCTCCAATCTTTCCTCCTCTCTTGGTTCCTT
CCATTCAGGCTGCAAACACAAAGTCTCTCCCATCTTAAACAAATTTCCTT
TCATCCCTGCTTCTCCATCAAGTGACGGTTCCAAGTCTCATTAGCAAAAG
CTGGGGCTGAGCGCCTACCACCTGTCAGGCCCTGGGCTTGGTGCTTGCTG
TGCCCATCTCATTTAACCCTGGTGATGACCCTATTGGGTAGGCACCATGG
ATACGATGAGGTAGCTGAAACTTGAAGACACCAGCGTCCTTGCCCAAGGT
 
CACAGGGTGGCAGACCCAACATTCCAACCCAGGACTATCTCCTCCCTAGC
CAGTGTGCTCTCACCAGTACTCGTATCTCCTTCTGTCTCTAGCAAACTCT
GAAGGAAGCATATATTGAGAAAGTGAGAGAAATCCAAATAAAGCTAGATG
AAGCAAAGAAAGGATAAATTTACATGACTAGGATGTGTGGGACTGGAGCT
GGCCTCTGAAGCTCTAGGACCTTGATGACACCCATGGTGTCTCTCGGCAA
GCACTCTCCCCATGTCCCTACTCTTCCTCTCTGTGTGCTGACCTCATTTT
GTCCTGAGACAGGCTTCCTCCCCGCAGTTGGTGGGGACAGGCCTTGGGG
GGAGGTGGCCACAGACAACTCTGCAATGACTTCATCCTCCAATGGGTTTA
GCAACCCAAGAGCTTTGCAGAACTGCTCTGTCTGAATGAATCTATACAGA
ATTGCAGGGAAGGCCTCTGATAGGCCTGGCTTTGGATAGGTGCCCACGGG
GGAAGGAGGAGAGGGCGGTGAACCCCCTCCTTCCAGCCCCAGGTTATGAT
AAAACACTTCCAACTAATTCTGCCCAAGGTCGCCTAGGAGGGGGTTGGTT
GATGTGACTGGCAGCCCTTCCAGAGTCATATGGTGGTGGGCAGTTCTCTA
AAGAAGTGACTGCCAATGTGAGAACCAAAGGGGAAGGGCTATTGGGCCCA
CGGAACCACAGGTGGCAGTCACAAGTGTCTGAGGCGGCATGGTGGGGAGG
GGCAGGCAGATAGAGGGGAGCCGGATTCTGAAGGGTGCTGGTTTCTGTAC
TGAGGAGTCTGCACTTTCTTCAGCAGGAAATTCACACATTCTCTCCCTCA
TTCATTCATTCACTCCCTCAGCAGGCGTTTCTCAGCGTCTGCGCTGAGCC
CAGCACTGTTCTGGGCACTGGGGCTCCAGAGGTGAGTAAATCCCTGCTGG
CCACTCTGGCCCTCAGCAGTCTGGTATAGAAGAAAAGAAACCTACAGTGT
TCCATCAGGTCTGAGGGGGCATCTGCAGGCCAGGGGAGGGGCGGGGCTAG
AGCTGGGCTTTTGGGGGAGCTGCCCCCAAGGCCCTTGCTGACCACCCCGC
TCCCGGCAGGCGCCTTCCTCGTGCCCTACTTCCTCATGCTGGCCATCTGT
GGCATCCCCCTCTTCTTCCTGGAGCTCTCCCTGGGCCAGTTCTCCAGCCT
AGGGCCCCTGGCTGTCTGGAAAATCAGCCCTCTCTTCAAAGGTGAGGCCT
CAGTGGTCCCCAGGGAGGGAAGGGCTCAGGGTCTGGGGGAGGCAGGGAGG
TTGCCCCCAGAACCCCTGCCAGCTCCAGGCAGAGGTGGAAGTGAAGCCCA
GATAGCTGCCAGCTCCCCAGAAGCCACTCCACCCGGCTGGCAGAGAGCTT
GGCCTGGGCAGCCCAGCAGCCTCTCCCCACACCAGGCGCCGGCGCAGCCA
TGCTGCTCATCGTGGGCTTGGTGGCCATCTACTACAACATGATCATCGCC
TACGTGCTCTTCTACCTCTTCGCCTCCCTCACCAGCGACCTACCCTGGGA
GCACTGTGGCAACTGGTGGAACACAGAACTCTGCCTGGAGCACAGAGTCT
CCAAGGACGGCAACGGGGCCCTGCCCCTCAACCTCACCTGCACCGTCAGC
CCCAGCGAGGAGTACTGGAGGTCAGGCAGCTGCTGGCCCCGCGGCATCTG
AGGGGACCCTGCACTGCTGAGGGTGGCCAGAGGGCATCCCCCACAGTCTC
AGCATGTACCGCCAAGATGAGCTCAGGTGGTGATGGCAGGTGGACTCTGG
CTTCTAATCCCAGCTCTTCACCTGACCAGCTGTGTGGCCTCAAACAATTT
ATTTGGCTTCTGTTGCTTTATCTGTAGCATGAGGGCAATAATACTAGGTA
TCTCAGAAAGGTTGTTGTGAAGATTAAAGGAGAGAAAGCATATATTAATA
AATTGTGCAGTCCAGTCTGGTGGATAGTAAGTGCTCAATAAATACTAGCT
GAGAATGATGATGATGATGATAACAGTAATAGTAACATTATGGAGTCTCA
GTTTCCTCCTCTGTAACAGGGGTTGGTAACAGTACCTGCTTCATAAGGTG
TTATCCAGTTAGTCATTTACCCACTCATTCATTCTTTTAATAAATATTTA
TTGAGCAACTGCTATATCATAGGTAAAGCACTTAGCATAAGGCCAGGACG
TACATAGTAAGGACCATCTCAGACTCCTCTAATAGAGATAGGACTATAAT
AGAGTAAGGGACTGTCATCAGAATTAAACAACATAATGCAGGTGAAACCA
TTATCACGGTGCTGGGCACACAGTGGGTCCTGGGAATGGTGGGTGTCGGA
AGGACGTTGCACTCATCAGCCTATCCCAATGCCCCATTTTATGGTCAATA
AAACAAAGCCTTGTCACTGCATTCTGCCTTTAAAAGAAGAAAGAGAAAGA
AAGAAAGAAAGAAAGAAAGAAAGAAAGAAAGAAAGAAAGAAAGAAAGAAA
GAAAGAAAGAGAAAGAAAGAAAGAAAGAAAGAAAGCAAAGCCCAGAGGCT
TGCTCAGTATTGCTCAGTAATTAGTGACAGAGATGGTATTGACACTAAGG
ACTCCTGAGTCCTGATCCTGTTCTGGAACATTCTAAAATAGCCTGGTCCC
TAGGTGGGACTGTTGGCTGGCCAGAGAACACAGACCCTATAACCCCCATC
CCTTACCTATAAAGGACTGCTGTTAGGAGATTTCTCCCAGATAGGCTCCG
TGTCTTGAAATGTTTTGGTCTTTAACCTGCCTGTAGTACATAGTTCATTC
ATTTGTCCACTTCAAGTCCATCTGAAGTGTCCGCACCTGCTGAAGAGTTT
ATCTGACCAGTGGGAAAGAATCAGCCTGAGTTTGCAGCTTAAAGCCAAAG
GATTTGATACAATCACTGGCAGCTGCCAGGGTGGTTCTCCATGGCCATGC
CACAGCCCACTGCATCAGTCAGGATGCAGGCAGGAGGCCGATGGATTGTC
AGAGGGTTTCACTGAAGGGAGTTTAGTGAAGGGACTTTTCAGAGGGTCAT
 
GGAAGCCACAGCAAAAAGTGAAGCATCAGTGACTAGCAACAGGGACAGCT
ATCACCACCCCTGAACCTAGGAGGCAATGGGAGGGAACCAGGAGAGAATC
AGAGCCTTGGAAGAGGGGCTACCTAAAGAACACAGTCATGGCCAGGCCCT
GGATGGTGTGTGTGTGTGTGTGTGTGTGTGTGTGTGTGTGTGTGTGTGTG
TGTGTGTGGTGTACACATGTATATGCAAATACCCAGACCCCTCATTCCTC
CCCTCACTACAGTCTCCCACTGTTGCCTCCCATTAGCCAAACCCACACGG
AAGCTGAAGAGGAAGGAAGAAGAGGAAGGGTGGTCTCGAACTCCTATCCT
CAAGTGATCTTCCTTCCTCAGCCTCCCAAAGCTGAGGAAGGGGTAAGGTC
TGGTGAGGCCAGCCTCCTGGGTCAGAGTTGGTCGGAGAAGGATGGAGAGG
GATCAAGAGGGCGTCAAGTGGAGAACAGCAGTAGAGCCTTGTGGGCTGGA
CATGGCTTGACTCCATGTCTGTGGAGCCTGGTGGGGTGACCAGGGGACAC
TGAGACCTTTGTCTCCCACAGCCGCTACGTCCTCCACATCCAAGGCAGCC
AGGGCATCGGCAGCCCTGGGGAGATCCGCTGGAACCTCTGCCTCTGCCTG
CTGCTGGCCTGGGTCATCGTGTTCCTCTGTATCCTCAAGGGTGTGAAGTC
TTCGGGCAAGGTGAAGCCTGGGAGGCCCCGGAGGCCTGAGGGGCTGGATG
GGGTGAAGGCTTCCTGGAGAAGGCAGGGCTTGGACTGAGCATGAGAAGAT
GAAGAGACTTCAGCTGGGCAGAGGGGAAGGGAGAGAGCCTTGAGACTGGG
GGATGAGGGCTGCTTGAAGAGAGACATGGAGGTGAGGACGCACGGGGGCA
AGGCTGCTTTACTAGACTGGAAGCTCACCAAGGCAGGGTCTACATTCAGC
TGTATCCTGACCCAGTGCTTGGCACTTTGAATGAGCTCAGCAAGAGTTTG
TTGAACACTTAGAGTGTGAGTGCATGAGTTAGGGGGCTCTTTGAGATGCA
GGTGACCAGAACCCAGTTATCACACTCAGTTGGAAAGTCCAGAGGGTGTG
CTTCAGGCACAGCTGGGTCTAGGGGTTCAATCAGTGTCATCAGGAAGCTG
GTTCTCTGGGTCTGTCAGTAGTGCTTCCTTCCAGGATGGCTTCCTTCTGA
GGCTTCATTCTTCGTGAGGTCACCCTAGCAGCTCTAGGCGTACATCTAAG
CTTGGTAAAGGGTTGTCTTTCCCCAGTTTGCAACTATTTAGAATTGAGT
CTCATTGGATGAGCAGGGCTGTGCACCCATCCCTAAATTAATCCCTGTGG
CCCGGAGGATGGAATGCTTTTCTTGGCTGGGCGTGGGTCATGTAACCACC
CCTGGAGCTGGGAATAGAGTCAGCTCCATGGTGGGAAAGTCAGGTACTGT
TATCTGAAAAAGGTAAACTAGAGACTGGAAGACAAAGCCACGGATGTCTA
CTTTAGGTGATGAAGGGGTGGAAAGGACGATAAAGCCATTTTGAAAAAAC
TTGGGGCCGGAGGTGGTGGCTCAAGCCTGTAATCCTAGCAGTTTGGGAAG
CCGAGGTGGGTGGATCACCTGAGGTCAGGAATTCAAGGCCAGCCTGGCCA
ACATGGTGAAACCCTGTCTCTACTAAAAATACAAAAATTAGCCGGGCATG
ATGGCAGGTGCCTGTAATCCCAGCTACTCGGGAGGCTGAGACGGGAGAAT
CGCTTGAACCTGGGAGACGGTAGTTGCAGTGAGCCAAGATTGTGCCACTG
CACTCCAGCCTGGGAGGCTGAGTGAGACTCCGTCTCAAAAAAAAGAAAAA
AGAAAAACTCAGAAACCTTCAAGATGGCTTGCCCAATCATTGGTAAATCA
GAACTTGTTTCCTCTGCAAGGTGCAGACGATACCAGAAGTATCATTTACA
AGGAACAGAGCCATTTAAGACAAAAAAGACAGGGTAGAAACCATATAGAA
AGAGAGAAGAGGTTATGGTCAAAGCCAAGGCAGTGAGGACGCTGAGACTT
GATGGTGTTTGCTGAAGCGGTGGCTGGAGGTTTGGAAAAGAAGGAAAGA
GGGAGGAGTGAGTTTGGGAATGTTGAATGTCTGAGGATGATGCAGAGTGA
GTTTGCAGGGGCAGGGAGTGGACACGGGGAGGAGGAGGCTTTTATAACCA
ACAATGGCGGCCTGGACCAGGGGCTGCAGTGGACATGGCCTTGGGAAGCA
AGGTGGTGGTGCTTTGCTGATGCTGGGTGTGTGAAGGGAGGAGGGAAGCC
TCAAGGATGAAGGCCGGTTCCTGGCTTGGGCTACCCAAAGGCTGGGGAGG
CAAGTGACACAGATGAGTTTACAAGGAATGGCACTGAGTCAAGGTCCCCG
ATGCCATCAGCTCCTCACTTATCATCTCTCAGGTGGTGTATTTCACGGCC
ACGTTCCCCTACCTCATCCTGCTCATGCTGCTGGTCCGCGGAGTCACCCT
CCCAGGGGCCTGGAAGGGCATCCAGTTCTATCTCACCCCCCAGTTCCACC
ACTTGTTGTCTTCCAAGGTGAGCCCCTCAGGGCGGGGTGCAGAGGGAGGG
GCCAGGCCTGAGCTGGAGAGTGGAAAGAGGGCTCCCTGGGACACCGCAGT
ACCAGGCACTCTGCTCAGCCCTTCTTGCGTCTTTTTATTTATTATTATT
GTTATTTTTTTCTATTTTTAAATTGATAGGTAAAATTTTATGTATTTATC
ATGTACAACATGATGTTTTGAAGCATATGTACCTGGGGGAACTGTTAAAT
CTAGCTAATTAGCATATGTATTATCTCACATAATTATCATTTTTGTAGTG
AGAACACTTAACATCCACTCTCTTAGCATTCTCCAAGAATACGATATATT
GTCACTAACTGCATATTATACTTCATTTATTATTATTAACAATCATGGAA
ACACTATCCTCATTTTACAGAGGAATAAACTGAGGCTCAAAAGGGTTCTT
ATCAAGCTTACGGTCATACAGCTAGGGGATCGAAGAACAGGGATCCCAGC
 
CCAGATTTGTGTGACTGTAAACCCCAAGGAGGGCATTAGTGACAATGGTG
ATAACAATGGTACTGACAGCTAATGTTAATATTTATTGACTGGGACTGT
GCCTGACCTTGCCCTAGTGTTTTACATGCATGTGTGTAGTGATTTAACCC
ACCCTCCTCCCATGAAGTAGGCACTATTCCTATCCCCTCGTTACAGATAG
GGATCCTGCGGTCCAGAGGGGTGACCTGGCTTGTTCAGGCTGCACAGCTA
GAAAATGGTGAAGCTGGGGTCCAAATCCAGGTGCCAGGATTCCAGAAAGG
AAGTAAAGGGGTTATGCAGGCTTACTGCCAATACAAATCTCCTGCACACT
TTACATTAGGCTCTGTGTCCTTCAGAACTGGACCAAGCTTAGAGACCATC
TTCTAGATCCTGGGTGGGAAACTGAAGCCCGCAGAGGGGAAGGGACTTGC
CCATGTCCCACCGTGTAAATGTTGGGGGAGGCTTTGCTCCTGGTCCACGT
CACTTGTGAGCCAGGCTCATGACCACGCCATCCCTCGTGACCACGCCATC
CCTGGAGCTGTCACACCATCTTCATTCACTGCCTTCCTTCTCTGTCCTTG
ACCATCCGCACACCCTCCCTTTCCATCCTTCCCGGCACAGGTGTGGATTG
AAGCTGCTCTTCAGATCTTCTATTCCCTGGGTGTGGGCTTCGGGGGGCTC
CTCACCTTTGCCTCCTACAACACGTTTCACCAGAACATCTATAGGTCAGT
GTCCCACAGCCTCCCAGACCCTGGGGTCCAAAGCAGGGAGGAGAGTGGCT
GGCCAGGGAGGGCCTCAGAGGCTGAAAGGAAGGCCCACTCACCCTGGCCC
GCACCTGGACTTCTTCTGGTAGAGACACTTTCATCGTCACTCTGGGCAAC
GCCATCACCAGCATCCTGGCTGGCTTTGCCATCTTCTCCGTGCTGGGCTA
CATGTCTCAGGAGCTGGGCGTGCCTGTGGACCAAGTAGCCAAAGCAGGTG
GGCAGGCTGCCAGGCCTCAGTGGGGTGAGCATGTGTGTTGGGTAGAGATG
GGGCTGGGCCAGTGGACCAGCAAGATTATGGATGGGGGCCAGGCGCGGTG
GCTCATGCCTGTGATCCCAGCACTTTGGGAGGCCAAGGTGGGCGGATCGC
TTGAGGTCAGGAGTTCGAGACCAGCCTGGTCAACCTGGTGAGTCCCAGTG
TCTACTAAAAATACAAAAAGTAGCCGGGCGTGGTGGCACATGCCTGTAAT
CCCAGTACTCAGGAGGCTGAGGCAGGAGAATCGCTTGAACCCGGGAGGTG
GAGGTTGCAATGAGCCAAGATTGGGTCACTGCCCTCTAGCATGGGCAACA
GAGCAAGACTCTGCCTCAAAAAAAAAAAAAAAAAGATTGTGGATGGGTATG
TCATGTGTGTGCATATGCACACATGTACGTGCAAAGGAACATGTCCTAGC
TGGTCTCTGGTGGTCCATGTGCAAGGGGATGGGTGAGCAAACGAGTGTGC
ATTAACCCATGTCTGGAGGCCAATGAGTTTCTGTGCAAGATTGTCACATT
TTGTGCTTTGGTTGCACAAATAAGTCATATTGTGTGTGGAGGTGTCTATG
TGCAAGTGAAAAGGGTGAGAATGATCTTGTGAATCACTGTGTCTTTTGCC
AGTGGATGCATGTAAATGTGTGTGTTAGTGGGGTTATTTGTACCAATAAT
TGTATACATAAGTATATTTCAATGAGGTTTTATATTGGGGTCTTGTGTTT
GTTTATCAACCTATGTTTATCGAGCACCTACAATGTGCTGGTGTAGGTCC
TGGGAGTTAAGGAAGTATCAGAGGGTGCATTTAAGTGGGCATCTCTGGGT
TAGGCTAAACAAATGGCCCAAATGAGTGTAAGTGGCCGTGTGTGTCTGAG
TGTGCGTATGGGAGCCCACCGCATGACCCAAGCTGCTGACCCCGTGTGCC
CCTGGCCCAGGCCCTGGCCTGGCCTTTGTCGTCTACCCACAGGCCATGAC
CATGCTGCCTCTGTCACCCTTCTGGTCCTTTCTCTTCTTCTTCATGCTTC
TGACTCTCGGCCTAGATAGCCAGGTGAGTCTCGTCTGTGGCAGCAGGCAC
CCCGTGTGTGTGTGGTGTGTGTGTGTGTGTGTGTGTGTGTGTGTGTGTGT
GTGTGTGTTGGGGATAGAATTCTGACCCCCAGCCCCTCCTCTCTCCTCAG
TTTGCTTTTCTGGAGACCATTGTGACAGCTGTGACAGATGAGTTCCCATA
CTACCTGCGGCCCAAGAAGGCGGTGTTCTCAGGGCTCATCTGCGTGGCCA
TGTACCTGATGGGGCTGATCCTCACCACTGATGTGAGTGGCGCTACAGGG
AGGATGGCAGGTGGGCGGACAAGGGCAGACGCCTGCAACGAGATCTCTG
GCCCAGCTGAGCAGTTGCTGGGCCCCCTCCATCTTCCTCTTTGCAAGGAA
CCCAGTCCCTCCCCGGCCCTATCTCCCAAGGGAGGCAGTTTGGCCTTGTG
ATCCAGAGTCCTGGCTGTGGAGATTTAGTCTGCAAGATCTGGGTTCAAAC
CTGACTCCTGCACTTGCTGAGGGGGCTTCAGGATAAATGATTTCCCCTCC
CTGAGATTGTTTTTCCTCATCAGTAAAAGGAGGGTTCTATAATAGACGGC
TGTCTTGGGACTCAGATAAGACAAGGCCTAGAAGGGCTGGGCTGGCAGCC
AGTGGTCACCCTGTCATCTTGTGCTGTCAGCATGGCTGCTTCCTGCTCAC
TTCTTGCCAGGAGAAGGGGCTGTAGCAGAGAACGAGGCCCAGGAAGGGGA
CACCAGAACTGTGCTTGTGTTTTAGGGGGGCATGTACTGGCTGGTCCTTC
TGGATGACTACAGCGCCAGCTTCGGGCTGATGGTGGTGGTTATCACCACG
TGCCTTGCCGTGACACGGGTGTATGGTGAGAAGAGCCGTGGGAAGTGGAG
TCGAGCTCTCCGCAGTGGGAGAATGGGAGTCTACCCTGGGGAGCCCCAGT
ACCTGGGTCCCTGGTCCCAAGGGCCAGGGTTTCCAGTGGGGGCAGCTGGG
 
GTAGAGGAGGGGCTGCTGGCGTTTGTGGGGCCGGCGGGTGGGGCCATTCC
TCCTCCCCTCCCCTGTGCAGGCATTCAGAGGTTCTGCCGAGACATCCACA
TGATGCTGGGCTTCAAGCCGGGCCTCTACTTCAGGGCCTGCTGGCTGTTC
CTGTCCCCAGCCACGCTCTTGGTAACTGGGGAGGGCGGAGGGTTTCTGG
CTGGGGCCCCAGAATTGAAAGCGGGAGTCCCCTCTGCACGTTCAGTCTGG
TAGGGCCACCCTGGCCCGCAGTGGAGGGCTGTGGGGTGGCCACCAGAATC
CTAGTCCATCCCCTCCCCTGATGTTCCCAGCTTCTGTAGCTGTGCTGGGC
CTGGCCCTAGTTCAGTTGGTTAAATGCGTGGGTCTTTCCCAGGCCCCAGG
GCCTCAGGATTGGAGCCTTTAGGCTGTGATTTCTGTCTTGGGGTTAGAGC
AGCTAAGGGTCTGGGGTGCCTCAGAAATGGCTTGTGCAGGCCTTGGTGTT
GGGTCAGCTTCAAGATGGCTGGCTAAACCCTCAAAGGCTCCAAAGATGG
CCCACTTCAGGGTTCGGTACAGCTTAGGATCCTGGGTAGCTCTGGGCTGG
ACTGTCTCAGGCCTTGGGCTGGCACTAGGCTGGGCTACCTCGGGCCTTAA
GCAGTTTAGACAAAAGCCCGCAGTGATGCTGGGAGTCCCCACTCTGCAGG
CCCTCATGGTGTATAGCATCGTCAAGTACCAGCCCTCGGAGTATGGCAGT
TACCGCTTCCCGCCCTGGCTGAGCTGCTGGGCATCCTGATGGGCCTGCT
GTCCTGCCTCATGATCCCAGCTGGCATGCTGGTGGCTGTGCTTCGAGAAG
AGGGCTCACTCTGGGAGGTGAGTCTGCCCACCCTGTCCACTCTCAGCCTT
CCTGACCTGTGGCCTGACCCTAGGTCCCCCTGCTAGAACAGAAAACATAT
GCACCCACCCCTTATCCAGCTTCTTTAGCCTGAGGAGACTTGCCTGGGC
TGAGGCAGATTCAGGGGCTGGAGTCCTGCCCTGTGGCACTTTGTACCACA
TGAAGCCTTAGGGGAGTGGGTGGCACAATCTAAAGACCACTGTTAACCAG
GAACTGGCCCCAGGTCCCACCGTGGGTTGGGGGTGTGTCAGGTCTTGAAC
TCAGGTCACCAGCCCAGAGCTTGCTTCTGGAACTCAGGGTCAAATGGCGA
CAGTGAGGGACCCCAGAGGGCAGGCTATCACCCTACTCCCGTGCCCAGGA
GTCCTGCTGTGTCCCACTGGTGGCCCGGGATAGAATGATGGTGTGAGCCC
TGCCCACGCCTAGGGTTTTGGGGAGGGACTCAGACAAACAGAGAGAGTGC
ACTCTGCCACAGGGTCTGAGGCCTTGGAAGAGAGGAGTAGTGACCCTTTG
TGGGCCAGCAGAAGTGGTGTGGTCTGAGCCTCATTGCACCCTCTGAACAC
TGAGGCCAGCACTTCTGCCGCCGCTGGTGTGGAGTCCTCCGGTGGCAAGA
GACCATCATCATGAATGCAGCTTTAGTGAGCCCGATGGATGGCATCCAGA
GAGAGCCTGGAGGCACCCACAACATACACATCCCACCAGAAGTGTGCTGG
AGCCAGCTAGCACTGGTTCATCCTTAAATATTCAGGAATGTGTGAGCTAG
GTGTTCAACCATTGGTAGCTAGGAATTGGCAATGGTGGAGATATTTACAC
CACAGAAAATGGCTACAAAGCGGGCCTCCCTTGCACCCCACCCCACAGTC
TGCTTACCAGCACACTACTGCCTCTCCTCCACTTCCCCCGTGTAGCTCCT
GCCCCTCAGCTCCCCCACCACAAGGCTCCACCTGCAGGGGTTGCTCAGGA
GGCAGAAGTGGTTTATGCCTCCCCCATCCTGCTGCCAGACCACAGGGGCA
CCCTGGAGTCTAGCCTCTGCACTGCCTCCAATTGCTGTGTGACTTGGGGC
AAACTCCTTCCCTTCTCTGAACCTCAGGAGCTCCACCTCTGCTCCTTGCT
TGAGAGCCAGGGTACCTGCAAAGGGAGGGAAGAGAGGGGAGTTGCTTAGG
GGGGAGGCTCCAGCTCCCAGGAAGCTCATGGTCCCCAAGCCTTGCTAATG
AGCCACTAGGAGTGCTGGGCTCTCCAGCAGTCACCACCGCCCCGCTGCCC
TGCTGCCCTGCTGCCCTGTCCCACAGCCAGGTGGAAGGGGCATGTTTTGG
GGGGCTCGAGAGCTGTGGTGAGCACTGCGGAAGAGGCATTCCCCAGCTCA
GGAATGCCAGCTTCAGAGGCCAGGGCTTCCAGCACTGACCCTAGGATCAG
AATCCTGCCTCTGCCATTTGCCAGCCTCGCGGCCCTAAGCAAGTTACTGC
ACTTCCCTGAACCTCAGCTTCTCTGTCTTTATTTTATTTTATTTTATTT
ATTTTTTTTGTTGAGACAGAGTGTTGCTCTGTCACCCAGGCTGGAGTGCA
GTGCAGTGGAGACAGTAAGCATAGTATCCAATAGGCAGGTTTTTTTGTCT
TTTTTTGTTTGTGTTTTGAGACAGAGTTTCACTTTGTCACCCAGGCTGGG
GTGCAGTGACGCCATCTGGGCTCACTGCAACCTCCGCCTCCCGGGTTCAA
GTGATTCTCCTGCCTCAGCCTCCCAAGTAGCTGGGATGACAAGCACCTGC
CACCACGTCCAGCTAATTTCTGTATTTTTAGTAGAAACAGGACCAGGCTG
GTCTTAAACTCCTGGCCTCAAGTGATCCGCGCACTTCGGCCTCCCAAAGT
GCTGGGATTAAGTGTCTTTGTCTTTAAAATGGAGAGAGTGATACCTACTT
TTAGGCCTGTTATGAGAAGAAAAGAAGTTTATCAATATAGTCTGCATTGT
GAAAGGCAGAAAAGCAAAATATTTTAAAGGTTCAGGATGGAGTCAGGACG
GGTTCAAATCCCAACGCCACCACTTCCCACCTGTGTGACCTTGGCCAACT
TAACTGATCTCTCTGAGCTTTAATACTCAGATAATATCAGTGCGGGCGGA
TGGTAAAAGTACTAATACCCCTCTCATTAGGACACTGTAAAGATTAAATT
 
AGCCAATGCTTAGCACAGAGCTGGAGTGCACTGGGTGCTCAATTTTAGGC
CAGGGGTTCTCACAGGGGGTGATTTTGCCCTTGGGGGACATTTGGCAATG
TCTGGAGACATTGTTGGTTGTCGTGGAGGGGAGAGGGATACAAGGAGGGG
TGCTCCTGTCATCTAGTGGGCAGAGTTCAGAGATGCTGCTAAACAACCTT
CCATGCGCAGGACAGCCCCCATAACACAGAATCCTCTAGACCGAAATGTC
AGGAGCACTCAGGTTAAGAAACTGGTCTGAGCACCCCCGTTTTCAGCTGG
AAACAAACCTAAGTCCTTGTTCTCAGGGAGCTTGTATTTTATTGTGGGGG
ACAGGCAATAAAGAAATATGTAAAGGTAAGTTCTGCACTTAAAAAAAAAA
GGAGTACAGGAATAAATGGAGAGTGGTACTTTGGCCAGGATGGTCAGAGA
AGACCTCTCTGAGGAGGTGGCACCTGACTAACCGGACAGGGTGATCCATG
TAGGTATCTAGGGTAAGAGCATTCCAGGCAGAAAGGCAACACGTGCAAAG
GCCCTGAGGCTGGGGGCTGGCATGTTTAAGAACAGCATGGAGGCCAGTGC
TGCGGCTGGAGCAGAGGGGGTGGAGAGAACTGGTGGAGCTGATATCAGGG
AGGTAGGCAGGGGCCAGGAGCTGGAGGCCCTGGTGCACACTTTGGATTTC
ATTTCCAGTGAGATGTGCAGCCATTGGCAGGCTTCTGAGCAGTGGGGTAG
CATGCGTTCAACACATCTTCCTAAGTATCACTGTGCTGTGAGTACCGAGC
TGTGGGAGACCCCGGCTAAAGGCATCACACATGGTATCTCCCTCGGAACC
TGCCCAGTGGGCTGCCCAAATGTTAATTGCCTCCTTTCTTTAAGGACACA
GGGGCTCCCTCTTCTCCCTGGCATGACAGGCAGAGGGATTTGGGAATGTG
GTTGAGGAAGAAGGAACTCTGGAATCTCCAGGCTTAGTCATTATCTTAGT
CACCCCGACTCCGTCCATGGAAACTGGGGCTCTACAAGGAAAGGTCAGA
GGCAGAAGGAGGCAGGTCGGGGCTGGAAGAAACCGGAAAGCCAGCTGCA
AGCCCACCGCAGTTCTCCCGGTTCATGGCTCTTTTCCAAAAGCCCCCAGC
TCCTTAAGATTCAGCATTTAGGGGAAGGCTCCCGGATTGGCATTCAGACA
CTCTGAGGAGGGGCCCGCCGCGTAATTATAGCAACAGCCGCCACTTCCCA
GGCCTTACCCTGTTCCCAGCACCCTGCTAAGTGCGTCATCTCAGTGCCTT
CACAACAACCCTGTGACAGGACTGGGATTCCCGAATTACAGACCAGGAAG
CAGAGCACCCAGAGCCGAGCGGCATTGCCAAGAGCCATCCAGCTTGCAAG
TGGTAGAACAGGATTCTCACCCAGGCAGGTGGATCCCTCCCCTGGGGAAG
TCATAGTGCCCCCCACTTCCCCGCCAGGGGGCTGTGGCCAGGACTCCCCC
AGTGATGTGGTCAGGTGTTTGCTGGGTGTCTGGCCACAGTGAACCCTCCA
CCAGCGCTGCCTGTTTCCTGTTTTCACTGCTCTCGTTGCTTTGCTGCAGC
GGCTCCAACAGGCCAGCCGGCCGGCCATGGACTGGGGACCATCGCTGGAG
GAGAACCGGACGGGCATGTATGTGGCCACGCTGGCTGGGAGCCAGTCACC
AAAGCCACTGATGGTGCACATGCGCAAGTACGGGGGCATCACCAGCTTCG
AGAACACGGCCATCGAGGTGGACCGTGAGATTGCAGAGGAGGAGGAGTCG
ATGATGTGAGGCAGGAGGCAGGCGGGCAGAAGGCCCTGCCCGGGACCTCA
CAGTCCCTTCTTAGAAGCCTGCAAAGGTCAGCTGTGCCCTCTGGGATTCT
GAGAGGCT
SEQ ID NO: 2: Sequence of the SCL6A7 protein (NP_055043) 
1 mkklqgahlr kpvtpdllmt psdqgdvdld vdfaahrgnw tgkldfllsc igycvglgnv 61
wrfpyraytn gggaflvpyf lmlaicgipl fflelslgqf sslgplavwk isplfkgaga 121
amllivglva iyynmiiayv lfylfaslts dlpwehcgnw wntelclehr vskdgngalp 181
lnltctvsps eeywsryvlh iqgsqgigsp geirwnlclc lllawvivfl cilkgvkssg 241
kvvyftatfp ylillmllvr gvtlpgawkg iqfyltpqfh hllsskvwie aalqifyslg 301
vgfgglltfa syntfhqniy rdtfivtlgn aitsilagfa ifsvlgymsq elgvpvdqva 361
kagpglafvv ypqamtmlpl spfwsflfff mlltlgldsq fafletivta vtdefpyylr 421
pkkavfsgli cvamylmgli lttdggmywl vllddysasf glmvvvittc lavtrvygiq 481
rfcrdihmml gfkpglyfra cwlflspatl lallvysivk yqpseygsyr fppwaellgi 541
lmgllsclmi pagmlvavlr eegslwerlq qasrpamdwg psleenrtgm yvatlagsqs 601
pkplmvhmrk yggitsfent aievdreiae eeesmm
SEQ ID NO: 3 : Sequence of Probe used for genotyping SNP3 
ATGTTCTTAA AATCACGGGA Acttaatttt tagagattta tttaaagtat ttaggggtaa
aaagtcataa tatctataat ttccttcaaa ttagcataaa aatagataaa gcaaacagca
aaatgttaac acttgttaat tttaggtggt gggtatgtat ctatcacatt atagtatttt
tctatagttt tgaaactttt tatgaaaaaa aGTTAAAAAA AATgattgct tgaggccagg
agttcaagac cagcctgggc aacatagcat gacctctgct tcaaaaaaaa aaaaaaatta
actaggcatg gtggtggcta ctcaggaggc taaggcagga ggattgctta agcccaggag
tacgaggata cactgagcta tgatcatgcc actgtactcc agcctgggca acaaagcaag
acctcatctt tttaaaaaaa gtgaaaacaa aaaaaaCTCT GGAACTAATG GTGCCAAGGT
CCCTTCCAGA TACACCACTG
R
TGCATGGATG ACTAGTTATA GTCTCCTTCT TAAGGATGTC TGGAATTTCA TCCAGAGTCC
CCAAGTCAAT AGCATCCGGG AAGGGGCTCC ACAGTCTTTT CTTGATTATG ACCAGTAGCC
CCTAGGCCCA GATTGGAGAA AGCCACAAAG GTTTCAAATC TCAGCCATCT AAGTGTGCCT
TCTCCTTGAA GACTCAACCA
SEQ ID NO: 4 : Sequence of Probe used for genotyping SNP4 
GGGTGGGAGG CCCCCTTCTT CAGGTCCCAC TCCTGGCTGG GACACTGGCA TGGCCATTGA
TCAAGGCCTT GGTCTCACAG AGTGGCAGCC CCACCAGACA GGAGGGGCAT AGACTTTGAG
AGAGAAGGAA AATCCAAGCC ACAGTCCGGA TGGCCGATGG GAGGCAAGCC CCCAGCCCTC
TCCTATACTC ACCTGGTTCA
Y
GGTGGGAATA GAGGGCACTA GAGCTGGGCT GGGCTCCGGT GTTAGACATC GGGGGTCTTT
GTTCAGCCCC GCCTGCCTTC TGAGCTCCTC TGCACTGGGG ACCACATGGT ACAGCTGTAG
TTTCCCAGCC GGCACATACC CACGATGTCC TAGGGAGCAT GCAGAAGGTT AGCAGGCTGC
GGGCCACCTT CCCGTCCCTT
SEQ ID NO: 5 : Sequence of Probe used for genotyping SNP5 
GTGGCCTGGG AGACAGCGGG CATTCATTAG TAATGGTGGA CACCTGAGGC TTGGGAAAGA
ACCAGGGTTC CAAGAGTAGA GGGGGAGGGG CTGGTGAAAA GGCGGCTGGC CCGGACTGAA
TAGAGTTTTC TGATCTTCAA AAAAATATgc tctgtgaccc tagggtaact gcttcccctc
tttgggcccg cattgcccca tctgtaaact tggggaagtt gTCTACCAGA AAGAAATAAC
TCTGGTCAAG TAAGTTTGGG AAATCCTGCT GAAGCCACTT CTCTGATAGA GTCACAGTGG
GCCATTAGCA CATCAAAGTC TCTGACAAGC CCCACAGCAA AGAAACCCCT TGAATTTTCT
TTCATCTGGC CTTCCCCGTG CTTTGCTACC TAGGAGGCCC CTACTCACAC CTCC
S
AGCATCCCAC TGAACTCCAC ACACTCTGCA GTGCTGGGAT GTTCAGGCTG GAGGGCCTCA
CCTTCTCTGT GTCTCagtat ttagcagcag gttttgtcgt caaatggcct ggatttcagc
gtgactactt ccaaactgca agacttcagg caagttactt agcttcttgg agcctcggcc
 
tccgcatgtg tcaaatggaa ataaaatagt ctaactgagc ttagaattgt GTTTCGCTCA
GGGCCCAGCA CATAAGAAGT GCCTGTTTTT GAGCCACCTG CTCACGGGGC TGCTGCTGCA
TCCCAGTGTG AGAGGCTGGC AGGCGTGGCT GATGTTCTAC CCACCTGAAG GAGGA
SEQ ID NO: 6 : Sequence of Probe used for genotyping SNP6 
GTCGCACAGC AAAGACGACG CAGGggggtg ggctgttagt gttccttgcc caactgtgga
aagggagtcc cagagagggc aggtcgcttg cccaaggtca cgcagAAAGC CGAGGGTTTC
CGTCGCGAAC CCCCCTCCCC ACTCGCTCAG GGCTCTCCAA TCCGCAGCTC TGCGTGCGGG
GGCGCGCGCA TCCCCCCGCC GTCCGTCCGT CAGCTGTCTG TCTGGGTGTC TATGCGGGCG
CAGCAGTGCA CCCTTCCCCA GCCTCGGGCG CTGCGCAGGG ACAGACAAGG C
R
TTCGCAGCGC CCTGCCCGCG CTCCACGCCC GCAGCCGCCA GACGGCAGCG CCTGCGTCCG
TGCCCGCCCC AGCCGGTGCG CGGGAGCCGC GGGGGCAAAG GCGCAGTGGC CAGCGGACCA
TCTCTCGTGC CCTCGCTCTC TGCGCTCCGG GGCAGCTGAG CCCCGGCCAC CCGCTCTCCA
AGATGAAGAA GCTCCAGGGA
SEQ ID NO: 7 : Sequence of Probe used for genotyping SNP7 
GCCAGGAGCT GATTGCCGGG TGTGGGGGGT ATTGGAATAC CTGAGCGTTG AGCTGGACTC
ATCTGAGGGG TGGGGAGTGG GAGGCGGTCA TCCATACTGA AGCCGGCTCC CTGAGCCTGC
GGGAAGACTC TCCTCTTTCC TGCCTGCTCC CTCCCCGCCC CCTTAGCTTG CCTGCTGAGA
CCCCAGGCTG CCCCTCAGCA GGGCTGAAGG GAGGCAAAGA CAGGGAGGGG GCTATCGGAG
GCAGGAGGAT ATGATCAATG AAGATGGAAG CTGGTATGGG AGAGTGGC
Y
GTGGGCCCAG ACCTCAGGCT CTCCCTACCT TGCCTTGTGG GATTGGACCT CTCAGCCAAG
CTGTCAGGAT ATGGGGAGGG GGAATACTGG GGGACGTTTT CTGGCTTGTA CCAGTCCTAA
CTAAggagtc agatctcctg aatactattt ctgccgctgc cacttgcctg gtgactttgg
acaagttCAT TCTCTAACCT GAACCTCCTC TGGCTAAGCC CTAGCCTGGA GGCACCAAAA
CAGGCCCCAC TGGGTGTGTG AGGTGTGGGG AAGAATGCTA AAGGGCTGGT AGATGTGAGA
AGACTGTTCT CAGGGGCTGG TGTTATCAAC CTCCCTacat acacacacat tcacactcac
actcactcac acacacacTC ATTCATTCAA GTTCTCTGCT CTGGGGCA
SEQ ID NO: 8 : Sequence of Probe used for genotyping SNP8 
TGGCCTCCGC TCCCCTCTGG GCATGCATCT CCTCTCCAGG GACAGCAAGA TTATGACTTT
CTGA
S
ACAGATCACG GCTTAAGTAC CTTCTTCCTG CTCATGAGCC AAAATTCACA AAATTCACCC
AGCCCTCAGG CTCTCCCTAC CTTGCCTGGT GGGGTTTGAC CCCCCCAGCC AAGCTGTCAG
CATGCGGGGA GGGGGAGTAT TGGGGAAGTT TTCTGGCTTG TGACTGAAAA GTACCAGTCC
 
TAACCAAGGA GTCAGATCTC CTTGGTTCTT AGTAGGCCCC ATTTGAGCCA CGGAGCATCA
GTGGATTCCC TCCCTCAGGG GAACCCCCAG GTCCCTCCAA GTCCTCTCCT TTCTATGCTG
AGGGCTCCCA GCTTCTTTCT GTTGTCCTCC TATCACCTCT TTTCCTGAAC ACCCTCCAGC
CTGGCCACCT GCCCTTTGGA CATGCTCCAT ATTGTCTGCA TCTTTCTTAA AATATGGTGC
CCGGAACGGA GGAACAAAAC TCCAGCACTG GTGGGTGAAG GAGACCTGGC TTTCGCATCA
GGAAGCCTTG GGCTCCAGAC CTAGTTCTTC CACTCACCAA CCTGGTAGAA ACCTCGG
SEQ ID NO: 9 : Sequence of Probe used for genotyping SNP9 
GGCCTGGCTT TGGATAGGTG CCCACGGGGG AAGGAGGAGA GGGCGGTGAA CCCCCTCCTT
Y
CAGCCCCAGG TTATGATAAA ACACTTCCAA CTAATTCTGC CCAAGGTCGC CTAGGAGGGG
SEQ ID NO: 10: Sequence of Probe used for genotyping SNP10 
AGAGATAGGA CTATAATAGA GTAAGGGACT GTCATCAGAA TTAAACAACA TAATGCAGGT
GAAACCATTA TCACGGTGCT GGGCACACAG TGGGTCCTGG GAATGGTGGG TGTCGGAAGG
ACGTTGCACT CATCAGCCTA TCCCAATGCC CCATTTTATG GTCAATAAAA CAAAGCCTTG
TCACTGCATT CTGCCTTTaa aagaagaaag agaaagaaag aaagaaagaa agaaagaaag
aaagaaagaa agaaagaaag aaagaaagaa agaaagagaa agaaagaaag aaagaaagaa
agCAAAGCCC AGAGGCTTGC TCAGTATTGC TCAGTAATTA GTGACAGAGA TGGTATTGAC
ACTAAGGACT CCTGAGTCCT GATCCTGTTC TGGAACATTC TAAAATAGCC TGGTCCCTAG
GTGGGACTGT TGGCTGGCCA GAGAACACAG ACCCTATAAC CCCCATCCCT TACCTATAAA
GGACTGCTGT TAGGAGATTT CTCCCAGATA GGCTCC
R
TGTCTTGAAA TGTTTTGGTC TTTAACCTGC CTGTAGTACA TAGTTCATTC ATTTGTCCAC
TTCAAGTCCA TCTGAAGTGT CCGCACCTGC TGAAGAGTTT ATCTGACCAG TGGGAAAGAA
TCAGCCTGAG TTTGCAGCTT AAAGCCAAAG GATTTGATAC AATCACTGGC A
SEQ ID NO: 11 : Sequence of Probe used for genotyping SNP12 
TTCCTGACCT GTGGCCTGAC CCTAGGTCCC CCTGCTAGAA CAGAAAACAT ATGCACCCAC
CCCTTATCCA GCTTCTTTTA GCCTGAGGAG ACTTGCCTGG GCTGAGGCAG ATTCAGGGGC
TGGAGTCCTG CCCTGTGGCA CTTTGTACCA CATGAAGCCT TAGGGGAGTG GGTGGCACAA
TCTAAAGACC ACTGTTAACC AGGAACTGGC CCCAG
R
TCCCACCGTG GGTTGGGGGT GTGTCAGGTC TTGAACTCAG GTCACCAGCC CAGAGCTTGC
TTCTGGAACT CAGGGTCAAA TGGCGACAGT GAGGGACCCC AGAGGGCAGG CTATCACCCT
ACTCCCGTGC CCAGGAGTCC TGCTGTGTCC CACTGGTGGC CCGGGATAGA ATGATGGTGT
GAGCCCTGCC CACGCCTAGG GTTTTGGGGA GGGACTCAGA CAAACAGAGA GAGTGCACTC
 TGCCACAGGG TCTGAGGCCT T
SEQ ID NO: 12 : Sequence of Probe used for genotyping SNP14

GGGTTCACCC CTGTGCCAGA ACTAGAGAGT GGCTTGGCGC TGGCTTTCAC TGGAAGGGCA
CCAGAGGATG ATGGGAGCTG AAGAGAGGAG CGACACTCAC ATCATAGGCG CCGGCTTTGA
TCTGCTGGTA CAGGCGGTGC TGGTCCTCAT CCCAGAACGG GGGGTACCCA ACCAGCAGGA
TGTACAGGAT GACCCCTGGA
R
AGAGGACCAG AGAACCTTCA ACCCCCTGTG GGGAGGAGAC ATGGGGGGAG GGGAGTGATG
GGAAAGGAGA GAGGGGGCCC CAGAGGCCGG GGTGCAGCCA GGGGCACAAA AGGGCCTTAG
GATGAACTCA CCACAAGCCC ACAGGTCCAC AGGCTTCCCG TACGGGTCCT TCCGCAGCAC
TTCTGGGGAG AGATATCCAG

### REFERENCES

Asperger (1944) Die autistischen Psychopathen im Kindesalter. Archiv für Psychiatrie und Nervenkrankheiten, 2:217-250.
Bailey A, Le Couteur A, Gottesman I, et al. (1995) Autism as a strongly genetic disorder: evidence from a British twin study. Psychol Med, 25:63-77.
Bailey A, Phillips W, Rutter M (1996) Autism: towards an integration of clinical, genetic, neuropsychological, and neurobiological perspectives. J Child Psychol Psychiatry 37(1):89-126.
Baird G, Charman T, Baron-Cohen S et al. (2000) A screening instrument for autism at 18 months of age: a 6-year follow-up study. J Am Acad Child Adolesc Psychiatry, 39(6):694-702.
Burger R and Warren R (1998) Possible immunogenetic basis for autism. Ment Retard Dev Disabil Res Rev, 4:137-141.
Carney RM, Wolpert CM, Ravan SA et al. (2003) Identification of MeCP2 mutations in a series of females with autistic disorder. Pediatr Neurol, 28(3):205-211.
Chakrabarti S and Fombonne E (2001) Pervasive developmental disorders in preschool children. JAMA, 285(24):3093-9
Comi AM, Zimmerman AW, Frye VH et al. (1999) Familial clustering of autoimmune disorders and evaluation of medical risk factors in autism. J Child Neurol, 14(6):388-394.
Connolly AM, Chez MG, Pestronk A et al. (1999) Serum autoantibodies to brain in Landau-Kleffner variant, autism, and other neurologic disorders. J Pediatr, 134(5):607-613.
Crump FT, Fremeau RT, Craig AM (1999) Localization of the brain-specific high-affinity 1-proline transporter in cultured hippocampal neurons: molecular heterogeneity of synaptic terminals. Mol Cell Neurosci, 13(1):25-39.
Fatemi SH, Halt AR, Stary JM et al. (2002) Glutamic acid decarboxylase 65 and 67 kDa proteins are reduced in autistic parietal and cerebellar cortices. Biol Psychiatry, 52(8):805-810.
Folstein S and Rutter M (1977) Infantile autism: a genetic study of 21 twin pairs. J Child Psychol Psychiatry Allied Disciplines, 18:297-321.
Folstein SE and Rosen-Sheidley BR (2001) Nat Rev Genet, 2:943-955.
Fremeau RT, Velaz-Faircloth M, Miller JW et al. (1996) A novel nonopioid action of enkephalins: competitive inhibition of the mammalian brain high affinity L-proline transporter. Mol Pharm 49(6):1033-1041.
Galli A, Jayanthi LD, Ramsey IS et al. (1999) L-proline and L-pipecolate induce enkephalin-sensitive currents in human embryonic kidney 293 cells transfected with the high-affinity mammalian brain L-proline transporter. J Neurosci, 19(15):6290-6297.
Gillberg C (1998) Chromosomal disorders and autism. J Autism Dev Disord, 28(5):415-425.
Gillberg C and Wing L (1999) Autism: not an extremely rare disorder. Acta Psychiatr Scand, 99:339-406.
Gillberg C and Coleman M (2000) The biology of the autistic syndromes, 3rd edn London: MacKeith Press.
Hussman JP (2001) Suppressed GABAergic inhibition as a common factor in suspected etiologies of autism. J Autism Dev Disord, 31 (2):247-248.
Jamain S, Quach H, Betancur C et al. (2003) Mutations of the X-linked genes encoding neuroligins NLGN3 and NLGN4 are associated with autism. Nat Genet, 34(1):27-29.
Jayanthi LD, Wilson JJ, Montalvo J et al. (2000) Differential regulation of mammalian brain-specific proline transporter by calcium and calcium-dependent protein kinases. Br J Pharmacol, 129(3):465-470.
Jorde LB, Mason-Brothers A, Waldmann R et al. (1990) The UCLA-University of Utah epidemiologic survey of autism: genealogical analysis of familial aggregation. Am J Med Genet, 36(1):85-88.
Jorde LB, Hasstedt SJ, Ritvo ER et al. (1991) Complex segregation analysis of autism. Am J Hum Genet, 49(5):932-938.
Kanner L (1943) Autistic disturbances of affective contact. Nervous Child, 2:217-250.
Lander E and Kruglyak L (1995) Genetic dissection of complex traits: guidelines for interpreting and reporting linkage results. Nat Genet, 11(3):241-247.
Le Couteur A, Rutter M, Lord C et al. (1989) Autism diagnostic interview: a standardized investigator-based instrument. J Autism Dev Disord, 19(3):363-387.
Lord C, Rutter M, Le Couteur A (1994) Autism Diagnostic Interview-Revised: a revised version of a diagnostic interview for caregivers of individuals with possible pervasive developmental disorders. J Autism Dev Disord, 24(5):659-685.
Nelson KB (1991) Prenatal and perinatal factors in the etiology of autism. Pediatrics, 87(5 Pt 2):761-766.
Nilsson M, Waters S, Waters N et al. (2001) A behavioural pattern analysis of hypoglutamatergic mice-effects of four different antipsychotic agents. J Neural Transm, 108(10):1181-1196.
Purcell AE, Jeon OH, Zimmerman AW et al. (2001) Postmortem brain abnormalities of the glutamate neurotransmitter system in autism. Neurology, 57(9):1618-1628.
Renick SE, Kleven DT, Chan J et al. (1999) The mammalian brain high-affinity L-proline transporter is enriched preferentially in synaptic vesicles in a subpopulation of excitatory nerve terminals in rat forebrain. J Neurosci, 19(1):21-33.
Rodier P and Hyman S (1998) Early environmental factors in autism. Mental Retard Dev Disord Res Rev, 4:121-128.
Singh VK, Warren RP, Odell JD et al. (1993) Antibodies to myelin basic protein in children with autistic behavior. Brain Behav Immun, 7(1):97-103.
Smalley SL (1997) Genetic influences in childhood-onset psychiatric disorders: autism and attention-deficit/hyperactivity disorder. Am J Hum Genet, 60(6):1276-1282.
Steffenburg S, Gillberg C, Hellgren L et al. (1989) A twin study of autism in Denmark, Finland, Iceland, Norway and Sweden. J Child Psychol Psychiatry, 30(3):405-416.
Szatmari P, Jones MB, Zwaigenbaum L et al. (1998) Genetics of autism: overview and new directions. J Autism Dev Disord, 28(5):351-368.
Weizman A, Weizman R, Szekely GA et al. (1982) Abnormal immune response to brain tissue antigen in the syndrome of autism. Am J Psychiatry, 139(11):1462-1465.

## Claims

1. A method of detecting the presence of or predisposition to autism in a subject, the method comprising detecting the presence of an alteration in the SLC6A7 gene in a sample from the subject, wherein the alteration is a mutation of one or more bases, an insertion of one or more bases, or a deletion of one or more bases.

2. The method of claim 1, wherein the presence of an alteration in the SLC6A7 gene is detected by sequencing, selective hybridisation and/or selective amplification.

3. The method of claim 1 or 2, wherein the alteration in the SLC6A7 gene is a point mutation.

4. The method of claim 3, wherein the alteration in the SLC6A7 gene is a SNP in the SLC6A7 gene selected from the group consisting of SNP6, SNP7, SNP8, SNP9, SNP10, and SNP12 reported in Table 1 or a haplotype comprising one or more of said SNPs.

5. The method of claim 4, wherein the alteration in the SLC6A7 is allele C of SNP8 which is correlated with autism.

## Patentansprüche

1. Verfahren zur Detektion der Anwesenheit von oder der Prädisposition für Autismus an einem Subjekt, wobei das Verfahren den Schritt umfasst, die Anwesenheit einer Veränderung im SLC6A7 Gen innerhalb der Probe von dem Subjekt zu detektieren, wobei die Veränderung eine Mutation einer oder mehrerer Basen, eine Insertion einer oder mehrerer Basen, oder eine Deletion einer oder mehrerer Basen ist.

2. Verfahren nach Anspruch 1, wobei die Anwesenheit einer Veränderung im SLC6A7 Gen durch Sequenzierung, selektive Hybridisierung und/oder selektive Amplifikation detektiert wird.

3. Verfahren nach Anspruch 1 oder 2, wobei die Veränderung im SLC6A7 Gen eine Punktmutation ist.

4. Verfahren nach Anspruch 3, wobei die Veränderung im SLC6A7 Gen ein SNP im SLC6A7 Gen ist, wobei der SNP aus SNP6, SNP7, SNP8, SNP9, SNP10 und SNP12, wie in Tabelle 1 aufgeführt, ausgewählt oder ein Haplotyp mit einem oder mehreren der erwähnten SNPs ist.

5. Verfahren nach Anspruch 4, wobei die Veränderung im SLC6A7 Gen das Allel C von SNP8 ist, welches mit Autismus korreliert ist.

## Revendications

1. Procédé pour la détection de la présence de ou d'une prédisposition à l'autisme chez un sujet, le procédé comprenant la détection de la présence d'une altération dans le gène SLC6A7 dans un échantillon provenant du sujet, dans lequel l'altération est une mutation d'une ou plusieurs bases, une insertion d'une ou plusieurs bases, ou une délétion d'une ou plusieurs bases.

2. Procédé selon la revendication 1, dans lequel la présence d'une altération dans le gène SLC6A7 est détectée par séquençage, hybridation sélective et/ou amplification sélective.

3. Procédé selon la revendication 1 ou 2, dans lequel l'altération dans le gène SLC6A7 est une mutation ponctuelle.

4. Procédé selon la revendication 3, dans lequel l'altération dans le gène SLC6A7 est un SNP dans le gène SLC6A7 choisi dans le groupe constitué du SNP6, SNP7, SNP8, SNP9, SNP10, et SNP12 rapportés dans le tableau 1 ou un haplotype comprenant un ou plusieurs desdits SNPs.

5. Procédé selon la revendication 4, dans lequel l'altération dans le SLC6A7 est l'allèle C du SNP8 qui est corrélé à l'autisme.
